# EUROPEAN PATENT SPECIFICATION

(11) **EP 2 825 039 B1**
(45) Date of publication and mention of the grant of the patent: **23.08.2017**
(21) Application number: 13761444.2
(22) Date of filing: 14.03.2013
(51) Int. Cl.: A61K 31/19, A61K 9/00, A61K 9/08, A61K 47/02, A61K 31/192

(54) **INJECTABLE IBUPROFEN FORMULATION**
INJIZIERBARE IBUPROFENFORMULIERUNG
PRÉPARATION INJECTABLE À L'IBUPROFÈNE

(30) Priority: 16.03.2012 US 201213422761; 16.03.2012 US 201213422738
(43) Date of publication of application: 21.01.2015
(73) Proprietor: Cumberland Pharmaceuticals Inc., Nashville, TN 37203 (US)
(72) Inventor: VILA, Andrew, Nashville, TN 37221 (US); PAVLIV, Leo, Cary, NC 27519 (US)
(74) Representative: Michalski Hüttermann & Partner Patentanwälte mbB
(86) International application number: PCT/US2013/031529
(87) International publication number: WO 2013/138628

(56) References cited:
- US-A1- 2003 191 187
- US-A1- 2004 132 823
- US-A1- 2009 175 940
- LUIS C GARZÓN ET AL: "Temperature Dependence of Solubility for Ibuprofen in Some Organic and Aqueous Solvents", JOURNAL OF SOLUTION CHEMISTRY, KLUWER ACADEMIC PUBLISHERS-PLENUM PUBLISHERS, NE, vol. 33, no. 11, 1 November 2004 (2004-11-01), pages 1379-1395, XP019285275, ISSN: 1572-8927
- SHAW LANCE R ET AL: "THE EFFECT OF SELECTED WATER-SOLUBLE EXCIPIENTS ON THE DISSOLUTION OF PARACETAMOL AND IBUPROFEN", DRUG DEVELOPMENT AND INDUSTRIAL PHARMACY, NEW YORK, NY, US, vol. 31, no. 6, 1 July 2005 (2005-07-01), pages 515-525, XP009085125, ISSN: 0363-9045, DOI: 10.1080/03639040500215784
- DATABASE WPI Week 201157, Derwent Publications Ltd., London, GB; AN 2011-H84806 ZHANG Y -& CN 102 085 179 A (LUO J) 08 June 2011

## Description

### BACKGROUND OF INVENTION

The present invention relates to a pharmaceutical composition for oral or injectable (parenteral) use containing 2-(4-isobutylphenyl)propionic acid.

2-(4-isobutylphenyl)propionic acid, whose International Nonproprietary Name is ibuprofen, is a well-known anti-inflammatory drug having a molecular weight of 206.28. (Merck Index 12th ed., n4925, page 839). Originally patented in the 1960's, ibuprofen is now marketed generically, as well as under the tradenames of Motrin®, Advil®, and Nuprin® for the treatment of pain, inflammation, and fever, and patent ductus arteriosis.

Ibuprofen is readily available as the racemic mixture ((RS)-Ibuprofen) of the two enantiomers, (R)-Ibuprofen and (S)-Ibuprofen. Even though the (S) enantiomer is the biologically active form, most preparations contain the racemic mixture since the (R) enantiomer is converted to the active (S) form in-vivo. For simplicity, hereinafter the term "ibuprofen" will be used to indicate any one of the (R) enantiomer, the (S) enantiomer, or the racemate.

Although ibuprofen has many advantages over other analgesics such as aspirin and acetaminophen, it is very poorly soluble in water. Thus, certain dosage forms of ibuprofen, especially oral or injectable liquids, have been difficult to develop. Several U.S. patents have addressed this problem.

For example, U.S. Pat. No. 4,309,421 appears to describe water-soluble complexes of ibuprofen and phospholipids suitable for parenteral administration. U.S. Pat. Nos. 4,859,704 and 4,861,797 appear to describe the synthesis of alkali metal salts of ibuprofen for preparing a liquid ibuprofen formulation.

Other U.S. patents appear to address this problem by preparing an ibuprofen salt with a basic amino acid as the active pharmaceutical ingredient and then solubilizing the salt to produce a liquid dosage form.

For example, U.S. Pat. No. 5,200,558 appears to describe enhanced analgesic effects of S (+) ibuprofen as salts of L and D amino acids, including arginine, in various dosage forms, including as an injectable solution. U.S. Pat. No. 4,279,926 appears to describe the use of basic amino acid salts of propionic acids for relieving pain and treating inflammatory conditions. Similarly, U.S. Pat. No. 5,463,117 appears to describe the preparation of salts of ibuprofen with basic amino acids. Finally, U.S. Pat. No. 6,005,005 appears to describe a liquid composition for oral use containing ibuprofen and arginine.

However, the approaches described in the patents discussed above have, among others, the disadvantage of requiring the formation of a salt before solubilization, where the salt must be isolated and tested prior to producing the dosage form. Additionally, the ibuprofen formulations resulting from those processes have at least a 1:1 molar ratio of amino acid or base to ibuprofen. It is beneficial from both a cost and development point to not have to form a salt and isolate and test it prior to producing the dosage form. It is also beneficial in most cases to minimize the amount of non-active components, including salts, used in therapeutic products in order to minimize potential side effects. Furthermore, for injectable products it is beneficial to produce a liquid dosage form of ibuprofen having a pH similar to that of blood (pH 7.4). Finally, it is beneficial for an injectable and oral product to have similar pharmacokinetics to minimize the need for dosage adjustments.

U.S. 6,727,286 provides a pharmaceutical composition comprising an aqueous solution of arginine and ibuprofen, wherein the molar ratio of arginine to ibuprofen is less than 1:1, as well as a method of making the same. An embodiment of the '286 patent is a pharmaceutical composition comprising an aqueous solution of arginine and ibuprofen, wherein the molar ratio of arginine to ibuprofen is less than 1:1, and wherein the pH of the solution is less than about 7.8. Another embodiment of the '286 patent is a method of making a pharmaceutical composition comprising an aqueous solution of arginine and ibuprofen, wherein the molar ratio of arginine to ibuprofen is less than 1:1, and wherein the pH of the solution is less than about 7.8. Still other embodiments of the '286 patent are directed to methods of treating pain, inflammation, fever, and/or other conditions alleviated by ibuprofen comprising administering a pharmaceutical composition comprising an aqueous solution of arginine and ibuprofen, wherein the molar ratio of arginine to ibuprofen is less than 1:1, and wherein the pH of the solution is less than about 7.8.

The '286 patent covers the Assignee's present commercial formulation of ibuprofen injection for intravenous use, sold under the trade name Caldolor®. Caldolor® is indicated in adults for the management of mild to moderate pain and the management of moderate to severe pain as an adjunct to opioid analgesics, and is commercially available as a 400 mg/4 mL single-dose vial (100 mg/mL) and 800 mg/8 mL single-dose vial (100 mg/mL). Caldolor must be diluted prior to intravenous infusion to a final concentration of 4 mg/mL or less.

Lance et al. described solubility of ibuprofen in phosphate buffer in function of temperature and presence of excipients (DRUG DEVELOPMENT AND INDUSTRIAL PHARMACY, vol. 31, no. 6, pages 515-525).

CN 102 085 179 describes ibuprofen injection comprising ibuprofen and alkaline cosolvents from anhydrides or hydrates of sodium carbonate, sodium bicarbonate, sodium citrate, sodium citrate, sodium phosphate, sodium hydrogen phosphate, sodium tartrate, and sodium acetate, wherein each 1000 ml of ibuprofen injection composition comprises from 80-120 g of ibuprofen, and preferably 100 g of ibuprofen; 30-55 g of an alkaline cosolvent, and preferably 45 g of an alkaline cosolvent; the remaining balance being water for injection.

Appropriate diluents include 0.9% Sodium Chloride Injection USP (normal saline), 5% Dextrose Injection USP (D5W), or Lactated Ringers Solution. The recommendation for diluting both strengths is to dilute in 250mL of diluent.

### OBJECTS AND SUMMARY OF THE INVENTION

The invention provides a pharmaceutical composition as defined in the claims. It is an object of the present invention to solubilize ibuprofen during the manufacture of a ibuprofen solution suitable for injection, wherein the ibuprofen in the solution remains soluble at concentrations from about 100mg/mL to about 5mg/mL, and preferably to about 4mg/mL, without undergoing a phase transition, e.g., without turning hazy and/or precipitating.

It is another object of the present invention to utilize an ibuprofen solubilizing agent to solubilize ibuprofen during the manufacture of the pharmaceutical product instead of using a salt form of ibuprofen, where the ibuprofen solubilizing agent provides an ibuprofen solution in which the ibuprofen is soluble in the solution from a concentration of about 100mg/mL to a concentration of about 5mg/ML, and preferably to about 4mg/mL, without turning hazy or precipitating at intermediate concentrations between 100mg/ML and 5mg/mL.

It is another object of the present invention to provide a pharmaceutical composition comprising an aqueous solution of an ibuprofen solubilizing agent and ibuprofen, wherein the molar ratio of the ibuprofen solubilizing agent to ibuprofen is less than or equal to about 1:1, where the ibuprofen solubilizing agent provides an ibuprofen solution in which the ibuprofen is soluble in the solution from a concentration of about 100mg/mL to a concentration of about 5mg/mL, and preferably to about 4mg/mL, without undergoing a phase transition, e.g,turning hazy and/or precipitating at intermediate concentrations between 100mg/ML and 5mg/mL.

It is another object of the invention to provide a pharmaceutical composition comprising an aqueous solution of an ibuprofen solubilizing agent and ibuprofen which provides an effective dose of ibuprofen, e.g., from about 400mg to about 800mg, in a smaller volume of solution, e.g., 40 - 50mL (400mg dose) and 80mL - 100mL (800mg dose).

In accordance with the above objects and others, the present invention is directed in part to a pharmaceutical composition comprising an aqueous solution of an ibuprofen solubilizing agent and ibuprofen, the ibuprofen solubilizing agent being in an effective amount such that the ibuprofen in the solution remains soluble at concentrations from 100mg/mL to 5mg/mL, and preferably to about 4mg/mL, without undergoing a phase transition, e.g, turning hazy and/or precipitating. In certain preferred embodiments, the ibuprofen solubilizing agent is in a molar ratio to ibuprofen of less than or equal to about 1:1.

In preferred embodiments, the pharmaceutical composition is sterile filtered or terminally sterilized.

In certain preferred embodiments, the ibuprofen solubilizing agent is sodium phosphate or potassium phosphate, and the molar ratio of the ibuprofen solubilizing agent to the ibuprofen is from about 0.7 to about 0.9:1. The molar ratio of the sodium or potassium phosphate to ibuprofen is preferably about 0.9:1.

In further preferred embodiments, the invention is directed to a pharmaceutical composition comprising an aqueous solution of water for injection, ibuprofen and an ibuprofen solubilizing agent selected from (i) tribasic sodium phosphate, tribasic potassium phosphate, or a combination of tribasic sodium phosphate and tribasic potassium phosphate, in a molar ratio of ibuprofen solubilizing agent to ibuprofen greater than 0.65:1 to 0.9:1; or (ii) dibasic sodium phosphate, dibasic potassium phosphate, monobasic sodium phosphate, monobasic potassium phosphate, or a combination of any of the foregoing, together with an alkalinizing agent in an amount suitable to provide a solubilized ibuprofen solution when ibuprofen is added to the ibuprofen solubilizing agent/alkalanizing agent mixture in a ratio of ibuprofen solubilizing agent to ibuprofen from 0.8 to 1.1:1, such that the pH of the resultant aqueous ibuprofen solution is from 6.5 to 9, the aqueous solution remaining clear and colorless when diluted from an ibuprofen concentration of 100mg/ml to 1mg/ml. In certain preferred embodiments, the ibuprofen solubilizing agent is tribasic sodium phosphate, tribasic potassium phosphate, or a combination of tribasic sodium phosphate and tribasic potassium phosphate, preferably tribasic sodium phosphate, in a molar ratio of ibuprofen solubilizing agent to ibuprofen from 0.7:1 to 1.1:1. In other preferred embodiments, the ibuprofen solubilizing agent is dibasic sodium phosphate, dibasic potassium phosphate, monobasic sodium phosphate, monobasic potassium phosphate, or a combination of any of the foregoing, preferably dibasic sodium phosphate, together with an alkalinizing agent in an amount suitable to provide a solubilized ibuprofen solution when ibuprofen is added to the ibuprofen solubilizing agent/alkalizing agent mixture in a ratio of ibuprofen solubilizing agent to ibuprofen from 0.8 to 1.1:1, such that the pH of the resultant aqueous ibuprofen solution is from 6.5 to 9. Preferably, in embodiments where the ibuprofen solubilizing agent is dibasic sodium phosphate, dibasic potassium phosphate, monobasic sodium phosphate, monobasic potassium phosphate, or a combination of any of the foregoing, the pH of the solution prior to the addition of ibuprofen is about pH 11.5 to about 12.5, or preferably about pH 12. The pharmaceutical composition of any of the above embodiments is preferably sterile filtered or terminally sterilized.

The pharmaceutical compositions described herein (e.g., in the above paragraph) preferably remain clear and colorless when stored for at least about 12 weeks at 25°C.

The pH of the pharmaceutical compositions described herein (aqueous solution of ibuprofen) is preferably from about 7 to about 9. In preferred embodiments, the aqueous solution comprises ibuprofen in a concentration of 5mg to 15 mg/mL. In preferred embodiments, the pharmaceutical composition (aqueous solution) is contained in a pharmaceutically acceptable container in a volume of from 40mL to 100mL. The aqueous solution contained in the bag may have an ibuprofen concentration of from about 5mg/mL to about 10mg/mL or from about 8mg/mL to about 10mg/mL and the volume of the aqueous solution may be, e.g., 40mL, 50mL, 80mL or 100mL. The pharmaceutically acceptable container is preferably acceptable for intravenous use, and may comprise, e.g., a pre-filled bag made of a pharmaceutically acceptable polymeric material, glass bottle, or plastic bottle. In certain embodiments, the aqueous solution is contained in a bag made from a material selected from polypropylene, polyolefin and polyvinylchloride. The pharmaceutical preferably comprises a dose of ibuprofen from 400 mg to 800 mg. For example, the aqueous solution may be contained in a bag for intravenous administration at an ibuprofen concentration from 5mg/mL to 15 mg/mL and an ibuprofen dose from 400 mg to 800 mg.

The pharmaceutical composition may be stored in pre-filled bags, e.g., polyolefin or polyvinyl chloride (PVC) bags. The pharmaceutical composition may also be stored in a container selected from a bag, a vial, or a bottle. In certain embodiments, the container is made from a flexible material such as a plastic, polypropylene, polyolefin, or PVC, or another pharmaceutically acceptable polymeric material or glass.

The ibuprofen used in the compositions and methods of the invention may be (RS)-Ibuprofen, or (S)-Ibuprofen.

In preferred embodiments, the pharmaceutical compositions of the invention have a pH from about 6.8 to about 10.0, and in certain preferred embodiments from about 7.0 to about 7.8. In certain embodiments, the pH of the aqueous solution of ibuprofen is about 6.8, 7.0, about 7.2, about 7.4, about 7.6, about 7.7, about 7.8, about 8.0, about 8.2, about 8.4, about 8.6, about 8.8 about 9.0, about 9.2, about 9.4, about 9.6, about 9.8, or about 10.0.

In other embodiments, the invention is directed to a method of treating a condition chosen from pain, inflammation, fever, and/or patent ductus arteriosis, comprising administering to a patient in need thereof an effective amount of an aqueous solution an ibuprofen solubilizing agent and ibuprofen, the ibuprofen solubilizing agent being in an effective amount such that the ibuprofen in the solution remains soluble at concentrations from 100mg/mL to 5mg/mL without undergoing a phase transition, e.g., turning hazy and/or precipitating. In preferred embodiments, the pharmaceutical compositions of the invention have a pH from about 6.8 to about 10.0. In certain preferred embodiments, the pH of the aqueous solution of ibuprofen is from about 7.0 to about 7.8. The administration is preferably via intravenous injection, but can also be via intramuscular injection or orally.

In certain preferred embodiments, the dose of ibuprofen administered to the patient comprises from about 100 mg to about 800 mg of ibuprofen. In certain embodiments, the effective amount is about 400 mg of ibuprofen. In certain preferred embodiments, the effective amount of the aqueous solution comprises about 800 mg ibuprofen. In other embodiments, the effective amount is about 7.5 mg/kg of ibuprofen.

In certain embodiments of the invention, the condition being treated in the patient is pain. In other embodiments, the condition is inflammation. In other embodiments, the condition is fever. In other embodiments, the condition is patent ductus arteriosis. In further embodiments, the patient is a critically ill patient receiving at least one treatment selected from vasopressor support and mechanical ventilation. In certain embodiments where the patient is a critically ill, the patient is further being treated by one or more of the following: the patient is being administered large volumes of blood products; is undergoing dialysis; is receiving multiple antibiotics; and/or the patient has a pulmonary artery catheter or an arterial blood pressure catheter inserted.

In certain preferred embodiments, the method of treatment comprises administering the aqueous solution of ibuprofen intravenously from a bag made of a flexible material such as a plastic, polypropylene, polyolefin, and PVC, or another pharmaceutically acceptable polymeric material. In other embodiments, the aqueous solution of ibuprofen is contained, e.g., in a bottle or vial made from, e.g., plastic or glass.

In other preferred embodiments, the invention is directed to a method of treating one or more conditions chosen from pain, inflammation, fever, and patent ductus arteriosis comprising administering to a patient in need thereof an aqueous solution comprising an effective dose of ibuprofen together with an effective amount of an ibuprofen solubilizing agent selected from sodium phosphate and potassium phosphate, wherein the molar ratio of ibuprofen solubilizing agent to ibuprofen is from about 0.65:1 to about 0.9:1, preferably about 0.7:1 to about 0.9:1.

The invention is further directed in part to a pharmaceutical composition comprising an effective dose of ibuprofen together with an effective amount of an ibuprofen solubilizing agent in an effective amount such that the ibuprofen in the solution remains soluble at concentrations from 100mg/mL to 5mg/mL without undergoing a phase transition, e.g., without turning hazy and/or precipitating, and the concentration of ibuprofen in the aqueous solution is from about 1mg/mL to about 100mg/mL, the aqueous solution being contained in a bag, or a glass or plastic vial or bottle. In certain embodiments, the pH of the aqueous solution of ibuprofen is from about 6.8 to about 10.0. In certain preferred embodiments, the pH of the aqueous solution of ibuprofen is from about 7.0 to about 7.8. In further preferred embodiments, the aqueous solution is at a concentration of 10 mg/mL and is contained in the bag at a volume of 80mL or 40mL. In further embodiments, the aqueous solution contained in the bag has an ibuprofen concentration of 8mg/mL and is contained in the bag at a volume of 100mL or 50 mL.

In certain preferred embodiments, the invention is directed to a pharmaceutical composition comprising an aqueous solution of ibuprofen and an ibuprofen solubilizing agent selected from sodium phosphate and potassium phosphate, wherein the molar ratio of ibuprofen solubilizing agent to ibuprofen is from about 0.65:1 to about 0.9:1, preferably about 0.7:1 to about 0.9:1. In certain preferred embodiments, the ibuprofen solubilizing agent is sodium phosphate in a molar ratio to ibuprofen of about 0.9:1. In certain preferred embodiments, the ibuprofen solubilizing agent is potassium phosphate in a molar ratio to ibuprofen of about 0.9:1.

In any of the foregoing, the pharmaceutical composition may comprise an effective dose of ibuprofen, e.g., a dose selected from 400 mg and 800 mg.

With respect to the pharmaceutical compositions of the present invention, these compositions preferably are stable. In certain preferred embodiments, the pharmaceutical compositions of the invention show no detectable chemical degradation after incubation for one month at 40°C. In addition, or alternatively, the pharmaceutical compositions can be stored at ambient conditions in prefilled polyolefin bags and remains clear and colorless for at least about 12 weeks. In addition, or alternatively, the pharmaceutical compositions can be stored in a bag made from a pharmaceutically acceptable polymer for at least about 12 weeks at 4°C. In addition, or alternatively, the pharmaceutical compositions can be stored in a bag made from a pharmaceutically acceptable polymeric material for at least about 12 weeks at 25°C. Preferably, the pharmaceutical compositions remain clear and colorless when stored in a bag made from a pharmaceutically acceptable polymer and exposed to at least one freeze-thaw cycle.

In certain preferred embodiments, the aqueous solution comprises ibuprofen in a concentration from about 1 mg/mL to about 100 mg/mL. In certain preferred embodiments, the aqueous solution comprises ibuprofen in a concentration of about 10 mg/mL. In certain preferred embodiments, the aqueous solution contained in the bag is 80mL at an ibuprofen concentration of 10mg/mL. In other preferred embodiments, the aqueous solution contained in the bag is 40mL at an ibuprofen concentration of 10mg/mL. In certain preferred embodiments, the aqueous solution contained in the bag has an ibuprofen concentration of 8mg/mL and a volume selected from 100mL or 50 mL.

In certain preferred embodiments, the invention is directed to a method of treating one or more conditions chosen from pain, inflammation, fever, and patent ductus arteriosis comprising intravenously administering to a patient in need thereof an aqueous solution comprising an effective dose of ibuprofen together with an effective amount of an ibuprofen solubilizing agent selected from sodium phosphate and potassium phosphate, wherein the molar ratio of ibuprofen solubilizing agent to ibuprofen is from about 0.65:1 to about 0.9:1, preferably about 0.7:1 to about 0.9:1 and the concentration of ibuprofen in the aqueous solution is from about 1mg/mL to about 100mg/mL. In certain embodiments, the method further comprises administering an 800 mg dose of ibuprofen to the patient. In other embodiments, the method further comprises administering a 400 mg dose of ibuprofen to the patient. In certain embodiments, the ibuprofen solubilizing agent is sodium phosphate. In certain other embodiments, the ibuprofen solubilizing agent is potassium phosphate. In certain preferred embodiments, the pH of the aqueous solution of ibuprofen is from about 7.0 to about 7.8. In certain preferred embodiments, the molar ratio of ibuprofen solubilizing agent to ibuprofen is about 0.9:1.

The invention is further directed in part to a pharmaceutical composition comprising an effective dose of ibuprofen together with an effective amount of a tribasic phosphate salt as an ibuprofen solubilizing agent (e.g., sodium phosphate, potassium phosphate, or mixtures thereof) wherein the molar ratio of ibuprofen solubilizing agent to ibuprofen is from about 0.65:1 to about 0.9:1, preferably from about 0.7:1 to about 0.9:1 and the concentration of ibuprofen in the aqueous solution is from about 1mg/mL to about 100mg/mL, the aqueous solution being contained in a bag made from a material selected from polypropylene, polyolefin and polyvinylchloride. In certain preferred embodiments, the pH of the aqueous solution of ibuprofen is from about 7.0 to about 7.8. In further preferred embodiments, the aqueous solution is at a concentration from about 5mg/mL to about 15 mg/mL, preferably from about 5mg/mL to about 10mg/mL, and is contained in the bag, e.g., at a volume of 80mL or 40mL (e.g., for an aqueous ibuprofen solution comprising ibuprofen at a 10mg/mL concentration). In further embodiments, the aqueous solution contained in the bag has an ibuprofen concentration of 8mg/mL and is contained in the bag at a volume of 100mL or 50 mL.

Certain preferred embodiments of the invention are directed to a pre-filled bag for intravenous administration, comprising a pharmaceutically effective dose (e.g., 400mg or 800mg) of ibuprofen and an ibuprofen solubilizing agent selected from sodium phosphate, potassium phosphate and mixtures thereof, the ibuprofen solubilizing agent being in a molar ratio to ibuprofen from about 0.7:1 to about 0.9:1, and the concentration of ibuprofen in the aqueous solution being from about 5mg/mL to about 15mg/mL. The aqueous ibuprofen remains physically and chemically stable in the pre-filled bag for intravenous administration for time period suitable to provide a useful shelf-life of the product (e.g., 12 months, 24 months, 36 months, 48 months, 60 months).

The invention is further directed in part to a method of preparing an aqueous solution of ibuprofen suitable for intravenous injection, comprising adding the ibuprofen solubilizing agent to water, mixing until the ibuprofen solubilizing agent is dissolved to form an aqueous solution of the ibuprofen solubilizing agent, adding ibuprofen to the solution, and mixing until the ibuprofen is dissolved to form the aqueous solution of ibuprofen solubilizing agent and ibuprofen, such that the ibuprofen solubilizing agent maintains the ibuprofen soluble in the aqueous solution at concentrations from 100mg/mL to 5mg/mL without undergoing a phase transition, e.g., without turning hazy and/or precipitating. In certain embodiments the method further comprises optionally adding sufficient water to result in the desired concentration of ibuprofen. In certain preferred embodiments, the method further comprises filtration to remove any possible particulate matter and remove possible microbial contamination, rendering it sterile. The product may also be sterilized by means of terminal sterilization (heat or irradiation). Alternatively, the method may further comprise lyophilizing the ibuprofen/ibuprofen solubilizing agent to form a lyophilized powder. In certain further embodiments, the method further comprises adjusting the pH of the aqueous solution to a pH from about 6.8 to about 10.0, and in certain embodiments from about 7.0 to about 7.8, using, e.g., any of the methods described herein.

For the purposes of the present invention, the phrase "without undergoing a phase transition" means that the aqueous solution of ibuprofen and ibuprofen solubilizing agent does not turn hazy and/or there is substantially no precipitate in the solution.

### DETAILED DESCRIPTION OF THE INVENTION

While the aqueous formulations described in the '286 patent provide pharmaceutically acceptable aqueous solutions of ibuprofen, it has been found that there are several limitations to that product. One such limitation is that the ibuprofen contained in aqueous ibuprofen formulations which comprise arginine and ibuprofen at a molar ratio of 0.92:1 are soluble at a concentration of 100mg/mL to about 20 or 25mg/mL, but thereafter go through a phase transition where the ibuprofen is not soluble and precipitates out of solution, until the solution is further diluted to a concentration of approximately 4 - 5mg ibuprofen/mL. It has also recently been discovered that the product may precipitate in polyolefin IV bags.

The present invention overcomes the limitations of the aqueous ibuprofen solutions comprised of arginine as an ibuprofen solubilizing agent together with ibuprofen. Thus, the present invention is directed in part to pharmaceutical compositions comprising an aqueous solution of ibuprofen together with an effective amount of an ibuprofen solubilizing agent such that the ibuprofen in the solution remains soluble at concentrations from 100mg/mL to 5mg/mL without undergoing a phase transition, e.g., without turning hazy and/or precipitating. Suitable ibuprofen solubilizing agents include, but are not limited to, sodium and potassium phosphate, sodium and potassium carbonate, sodium hydroxide, and L-lysine, at molar concentrations relative to the ibuprofen such that the resultant aqueous ibuprofen solution remains soluble at concentrations from 100mg/mL to 5mg/mL without undergoing a phase transition. A further suitable ibuprofen solubilizing agent is L-arginine at a molar ratio to ibuprofen greater than about 1.05:1.

In preferred embodiments, the molar ratio of ibuprofen solubilizing agent to ibuprofen in the pharmaceutical composition is less than 1:1. Certain other embodiments of the invention are directed to pharmaceutical compositions comprising an aqueous solution of an ibuprofen solubilizing agent and ibuprofen which have a molar ratio of ibuprofen solubilizing agent to ibuprofen greater than 1:1, with the proviso that the ibuprofen in the solution remains soluble at concentrations from 100mg/mL to about 5mg/mL, preferably to about 4mg/mL, without undergoing a phase transition, e.g., turning hazy and/or precipitating. In preferred embodiments, the pH of the aqueous solution of ibuprofen is from about 7.0 to about 7.8.

Pharmaceutical compositions comprising an aqueous solution of ibuprofen and an ibuprofen solubilizing agent selected from sodium phosphate and potassium phosphate preferably have a molar ratio of ibuprofen solubilizing agent to ibuprofen from about 0.7:1 to about 0.9:1.

The present inventor has further discovered that a liquid composition of ibuprofen can be produced by combining ibuprofen with the ibuprofen solubilizing agents of the invention at molar ratios that minimize the amount of ibuprofen solubilizing agent(s) necessary to solubilize the ibuprofen, and that achieve a composition having a pH that is suitable for injection. Thus, another embodiment of the invention is a pharmaceutical composition comprising an aqueous solution of ibuprofen, wherein the pH is from about 6.8 to about 10.0. In yet another embodiment of the invention, the pharmaceutical composition comprises an aqueous solution of ibuprofen, wherein the pH is from about 7.0 to about 7.8. A further embodiment of the invention is a pharmaceutical composition comprising an aqueous solution of ibuprofen, wherein the pH is about 6.8, about 6.9, about 7.0, about 7.1, about 7.2, about 7.3, about 7.4, about 7.5, about 7.6, about 7.7, about 7.8, about 7.9, about 8.0, about 8.1, about 8.2, about 8.3, about 8.4, about 8.5, about 8.6, about 8.7, about 8.8, about 8.9, about 9.0, about 9.1, about 9.2, about 9.3, about 9.4, about 9.5, about 9.6, about 9.7, about 9.8, about 9.9, or about 10.0.

The present inventor has further discovered a method of making a pharmaceutical composition comprising an aqueous solution of ibuprofen and an ibuprofen solubilizing agent that maintains the ibuprofen soluble in the aqueous solution at concentrations from 100mg/mL to 5mg/mL without undergoing a phase transition, e.g., without turning hazy and/or precipitating, wherein the method comprises the following: adding the ibuprofen solubilizing agent to water, mixing until the ibuprofen solubilizing agent is dissolved to form an aqueous solution of the ibuprofen solubilizing agent, adding ibuprofen to the solution, and mixing until the ibuprofen is dissolved to form the aqueous solution of ibuprofen solubilizing agent and ibuprofen, optionally adding sufficient water to result in the desired concentration of ibuprofen. The pH of the resulting solution can be adjusted using techniques known in the art to achieve a desired pH, for example a pH similar to that of blood. The resulting product is a clear, colorless solution that can readily be passed through a 0.2 micron filter. Finally, the resulting solution can be terminally sterilized or lyophilized.

Alternatively, the ibuprofen can be added prior to the ibuprofen solubilizing agent, or the ibuprofen solubilizing agent and ibuprofen can be added at the same time. Moreover, the pH of the solution can be adjusted by adding additional ibuprofen solubilizing agent or ibuprofen to achieve the desired pH. For example, in one embodiment of the invention, an aqueous solution of ibuprofen solubilizing agent and ibuprofen is prepared that results in a molar ratio of less than 1:1, and then additional ibuprofen solubilizing agent is added to achieve a pH of about 6.8 to about 10.0, and in certain embodiments preferably a pH from about 7.0 to about 7.8.

The present inventor has further discovered a method of treating a condition chosen from pain, inflammation, fever, and/or other conditions alleviated by ibuprofen comprising administering to a patient in need thereof an effective amount of a pharmaceutical composition comprising an aqueous solution of ibuprofen solubilizing agent and ibuprofen, as described herein. Additionally, the present inventor has discovered a method of treating a condition chosen from pain, inflammation, fever, and/or other conditions alleviated by ibuprofen comprising administering to a patient in need thereof an effective amount of a pharmaceutical composition comprising an aqueous solution of ibuprofen solubilizing agent and ibuprofen, wherein pH of the aqueous solution from about 6.8 to about 10.0. Other conditions alleviated by ibuprofen include, but are not limited to, patent ductus arteriosis and certain forms of cancer. In certain embodiments, the patient is a critically ill patient receiving at least one treatment selected from vasopressor support and mechanical ventilation. In certain embodiments, the patient is a critically ill patient selected from the group consisting of a patient who is being administered large volumes of blood products; is undergoing dialysis; is receiving multiple antibiotics; has a pulmonary artery catheter or an arterial blood pressure catheter inserted; and combinations of any of the foregoing.

The pharmaceutical composition may be administered by injection (intravenous or intramuscular) or orally. Dosages of the pharmaceutical composition range from about 5 mg to about 1000 mg of ibuprofen in the pharmaceutical composition and can be determined by one of ordinary skill in the art. In one embodiment, the dosage is from about 100 to about 800 mg of ibuprofen in the pharmaceutical composition. In a further embodiment, the dosage is about 400 mg of ibuprofen in the pharmaceutical composition. In still another embodiment, the dosage of the pharmaceutical composition is from about 5 to about 10 mg/kg, and in a further embodiment the dosage of the pharmaceutical composition is about 7.5 mg/kg.

In certain preferred embodiments, the pharmaceutical composition (e.g., aqueous solution of ibuprofen) may include ibuprofen at a concentration of from about 1mg/mL to about 100mg/mL, and in certain preferred embodiments about 10mg/mL. The aqueous ibuprofen solution may be filled, e.g., at 80mL at 10mg/mL for a 800mg ibuprofen dose and 40 mL for a 800mg ibuprofen dose. Alternatively, an 8mg/mL ibuprofen concentration filled at either 100mL or 50mL is contemplated for a 800mg ibuprofen dose and a 400mg ibuprofen dose, respectively. Obvious variations of concentrations and volumes of ibuprofen providing pharmaceutically acceptable doses of ibuprofen would be contemplated by one having ordinary skill in the art, and are encompassed by the present specification and the appended claims.

Certain embodiments of the invention are directed to the aqueous ibuprofen solution in pharmaceutically acceptable containers, which may be sterilized prior to labeling and secondary packaging. The product can be sterile filtered or terminally sterilized. The containers may or may not have an overwrap, if needed to minimize water loss through the plastic. The product can be stored at ambient conditions and shipped to hospitals in prefilled bags. The container is preferably a bag made of polypropylene, polyolefin, PVC, or other pharmaceutically acceptable polymeric materials. The product can also be filled into glass or plastic bottles and infused into patients.

In certain preferred embodiments, the ibuprofen solubilizing agent is a tribasic phosphate, most preferably sodium or potassium phosphate. The tribasic phosphates solubilize ibuprofen at less than a 1:1 molar ratio. In contrast, dibasic and monobasic phosphates on their own do not solubilize ibuprofen. The tribasic phosphates have unique beneficial properties as compared to arginine, including the fact that aqueous solutions of ibuprofen which utilize tribasic phosphate salts as ibuprofen solubilizing agents as set forth herein do not go through phase transition and precipitate at ibuprofen concentrations between, e.g., 100mg/mL and 5mg/mL (or less), and are soluble at all ranges from the maximum solubility down through less than 4mg/mL. The product is also compatible with all IV bag materials with no evident precipitation, including forced freeze thaws. These are very important features with respect to containing an aqueous solution of ibuprofen as described herein in a pre-filled bag, e.g, at an ibuprofen concentration between 5mg/mL and about 15mg/mL, or between about 5mg/mL and 10mg/mL. The higher ibuprofen concentration in solution in the pre-filled bag, e.g., 8 to 10mg/mL (as compared to 3.24mg/mL typically used for the commercially available Caldolor® product) allows for significantly reduced volume (better patient safety, children, fluid restricted patients, etc), and allows such pre-filled bag products to constitute small volume parenterals.

In certain preferred embodiments, the ibuprofen solubilizing agent is dibasic sodium phosphate, dibasic potassium phosphate, monobasic sodium phosphate, monobasic potassium phosphate, or a combination of any of the foregoing, preferably dibasic sodium phosphate, together with an alkalinizing agent in an amount suitable to provide a solubilized ibuprofen solution when ibuprofen is added to the ibuprofen solubilizing agent/alkalanizing agent mixture in a ratio of ibuprofen solubilizing agent to ibuprofen from 0.8 to 1.1:1, such that the pH of the resultant aqueous ibuprofen solution is from 6.5 to 9. Preferably, in embodiments where the ibuprofen solubilizing agent is dibasic sodium phosphate, dibasic potassium phosphate, monobasic sodium phosphate, monobasic potassium phosphate, or a combination of any of the foregoing, the pH of the solution prior to the addition of ibuprofen is at least pH 12.

The alkalinizing agent may be chosen from alkali or alkaline-earth metal hydroxides, carbonates, bicarbonates and phosphates, sodium borate as well as basic salts of organic acids (example: sodium citrate).

Exemplary alkalinizing agents may include sodium hydroxide, potassium hydroxide, calcium hydroxide, magnesium hydroxide, ammonium hydroxide, sodium carbonate, potassium carbonate, magnesium carbonate, sodium bicarbonate, potassium bicarbonate, sodium tetraborate, sodium citrate, potassium citrate, sodium acetate, potassium gluconate, sodium sulfite, sodium phosphates, potassium phosphates, hydrogen orthophosphate, sodium aluminate, magnesium aluminate, dibasic ammonium phosphate, magnesium oxide, magnesium hydroxide, tris(hydroxymethyl)aminomethane, monoethanolamine, diethanolamine, alkylene diamines (such as methylene diamine, ethylene diamine, or propylene diamine or combinations thereof), amino alcohols (such as methanolamine, monoethanol amine, or propanolamine or combinations thereof), meglumine, arginine, lysine, ethane, and the mixtures of any of the above.

### DETAILED DESCRIPTION OF PREFERRED EMBODIMENTS

The following examples represent specific embodiments of the foregoing discovery, but are not representative of the entire scope of the invention. The ibuprofen and ibuprofen solubilizing agents used in the examples are United States Pharmacopeia grade, but other pharmaceutically acceptable ibuprofen solubilizing agents can be utilized.

### EXAMPLE 1 - Manufacture of Ibuprofen/Na₃PO₄ Solution

An ibuprofen formulation comprising sodium phosphate (Na₃PO₄) in a molar ratio of phosphate salt to ibuprofen of 0.9:1 is prepared as follows:
1. 7.15 kg of Na₃PO₄ are added, mixed and dissolved in 125 kg of water for injection.
2. 10 kg of ibuprofen are added, mixed and dissolved in the sodium phosphate solution from step 1.
3. 3.5 kg of sodium chloride are added, mixed and dissolved in ibuprofen/phosphate solution from step 2.
4. Water for injection is added to achieve a final weight of 1000 kg.
5. The solution is passed through a 0.2micron filter and filled into the appropriate container.
6. The containers may be terminally sterilized prior to labeling and secondary packaging.

### EXAMPLE 2 - Manufacture of Ibuprofen/K₃PO₄ Solution

An ibuprofen formulation comprising potassium phosphate (K₃PO₄) in a molar ratio of phosphate salt to ibuprofen of 0.9:1 is prepared as follows:
1. 9.26 kg of K₃PO₄ are added, mixed and dissolved in 125 kg of water for injection.
2. 10 kg of ibuprofen are added, mixed and dissolved in the sodium phosphate solution from step 1.
3. 2.5 kg of sodium chloride are added, mixed and dissolved in ibuprofen/phosphate solution from step 2.
4. Water for injection is added to achieve a final weight of 1000 kg.
5. The solution is passed through a 0.2micron filter and filled into the appropriate container.
6. The containers may be terminally sterilized prior to labeling and secondary packaging.

### EXAMPLE 3 - Solubility of Ibuprofen with Sodium and Potassium Phosphate

Ibuprofen (IBU) was completely dissolved at 70 mg/mL when sodium phosphate tribasic (Na₃PO₄) (Table 1) was used and up to 100 mg/mL when potassium phosphate tribasic (K₃PO₄) (Table 2) was used. The molar ratios of PO₄ to IBU were 0.6:1, 0.7:1.0, 0.8:1.0, and 0.9:1.0 for Na₃PO₄:IBU, and 0.7:1.0 to 0.9:1.0 for K3PO4:IBU. The IBU solutions were all clear and colorless at each concentration tested with both Na₃PO₄ and K₃PO₄ (Tables 1 and 2), except for the 0.6:1 ratio for Na₃PO₄:IBU. The 0.6:1 ratio for Na₃PO₄:IBU provided a visible precipitate. Injectable formulations should not have insoluble, particulate matter contained therein, and therefore that ibuprofen formulations did not provide an acceptable product. Each of the other IBU formulations of Example 3 provided an acceptable product.

**Table 1: Solubility of IBU with Na₃PO₄**

| | Na₃PO₄ | | | | | | | |
|---|---|---|---|---|---|---|---|---|
| Molar Ratio | 0.6:1.0 | | 0.7:1.0 | | 0.8:1.0 | | 0.9:1.0 | |
| Concentration (mg/mL) | Appearance | pH | Appearance | pH | Appearance | pH | Appearance | pH |
| 70 | Visible precipitate | NA | Clear and colorless | 7.21 | Clear and colorless | 7.53 | Clear and colorless | |
| 16 | NA | NA | Clear and colorless | 7.07 | Clear and colorless | 7.40 | Clear and colorless | 7.42 |
| 8 | NA | NA | Clear and colorless | 7.13 | Clear and colorless | 7.52 | Clear and colorless | 7.35 |
| 4 | NA | NA | Clear and colorless | 7.19 | Clear and colorless | 7.60 | Clear and colorless | 7.33 |

**Table 2. Solubility of IBU with K₃PO₄**

| | K₃PO₄ | | | | | |
|---|---|---|---|---|---|---|
| Molar Ratio | 0.7:1.0 | | 0.8:1.0 | | 0.9:1.0 | |
| Concentration (mg/mL) | Appearance | pH | Appearance | pH | Appearance | pH |
| 100 | Clear, colorless | 7.13 | Clear, colorless | 7.34 | Clear, colorless | |
| 25 | Slight, White haze | 6.88 | Clear, colorless | 7.02 | Clear, colorless | 7.20 |
| 10 | Clear, colorless | 6.74 | Clear, colorless | 7.07 | Clear, colorless | 7.27 |
| 8 | Clear, colorless | 6.77 | Clear, colorless | 7.09 | Clear, colorless | 7.37 |
| 4 | Clear, colorless | 6.83 | Clear, colorless | 7.16 | Clear, colorless | 7.38 |
| 3.2 | Clear, colorless | 6.85 | Clear, colorless | 7.18 | Clear, colorless | 7.40 |

### EXAMPLE 4: Physical Stability of Ibuprofen Formulations Containing Sodium Phosphate and Potassium Phosphate

Ibuprofen ("IBU") formulations containing Na₃PO₄ and K₃PO₄ were further tested for physical and chemical stability. First, the physical stability of Ibuprofen/phosphate formulations ("IBU:PO₄) at different ibuprofen concentrations (4mg/mL, 8 mg/mL and 16 mg/mL) with the ibuprofen:phosphate in a molar ratio of 1.0:0.85, in IntraVia® IV bags from Baxter was tested. The results are set forth in Table 3 below:

**Table 3: Physical Stability of IBU:PO₄ formulations at 4, 8, and 16 mg/mL**

| Sample | Conc. (mg/mL) | Freeze-Thaw Cycles | | | 4°C | 25°C |
|---|---|---|---|---|---|---|
| | | 1 | 2 | 3 | 12 weeks | 12 weeks |
| IBU:K₃PO₄ (1.0:0.85) | 16 | Clear, colorless | Clear, colorless | Clear, colorless | Clear, colorless | Clear, colorless |
| | 8 | Clear, colorless | Clear, colorless | Clear, colorless | Clear, colorless | Clear, colorless |
| | 4 | Clear, colorless | Clear, colorless | Clear, colorless | Clear, colorless | Clear, colorless |
| IBU:Na₃PO₄ (1.0:0.85) | 16 | Clear, colorless | Clear, colorless | Clear, colorless | Clear, colorless | Clear, colorless |
| | 8 | Clear, colorless | Clear, colorless | Clear, colorless | Clear, colorless | Clear, colorless |
| | 4 | Clear, colorless | Clear, colorless | Clear, colorless | Clear, colorless | Clear, colorless |

The results show that each of the formulations remained clear and colorless under each of the conditions tested, i.e., 1-3 freeze-thaw cycles, and storage at 4°C for 12 weeks and storage at 25°C for 12 weeks.

Ibuprofen ("IBU") formulations containing Na3PO4 were further tested for physical and chemical stability at an ibuprofen concentration of 10 mg/mL with the ibuprofen:phosphate in a molar ratio of 1.0:0.9 in different types of IV bags: IntraVia®(Baxter), Technoflex® ( Technoflex), PAB®(Braun), and Grifols ® (Grifols). The results are shown in Table 4.

**Table 4: Physical and Chemical Stability in Different Types of IV Bags**

| **Sample** | **IV bag** | | **Freeze-Thaw Cycles** | | | **Particle** | **Analysis** | **pH** |
|---|---|---|---|---|---|---|---|---|
| | | Sample | 1 | 2 | 3 | 10 µm | 25 µm | - |
| 10 mg/mL IBU:Na₃PO₄ | IntraVia® | 1 | Clear, colorless | Clear, colorless | Clear, colorless | 3.6 ± 0.40 | 0.20 ± 0.20 | 7.53 |
| (1.0:0.9) | | 2 | Clear, colorless | Clear, colorless | Clear, colorless | - | - | 8.04 |
| | Technoflex® | 1 | Clear, colorless | Clear, colorless | Clear, colorless | 4.47 ± 0.70 | 0.67 ±0.12 | 7.50 |
| | | 2 | Clear, colorless | Clear, colorless | Clear, colorless | - | - | 8.01 |
| | PAB® | 1 | Clear, colorless | Clear, colorless | Clear, colorless | 13.53 ± 1.50 | 0.47 ± 0.12 | 7.45 |
| | | 2 | Clear, colorless | Clear, colorless | Clear, colorless | 8.07 ± 0.23 | 0.27 ± 0.31 | 7.95 |
| | Viaflex® | 1 | Clear, colorless | Clear, colorless | Clear, colorless | 10.67 ± 1.79 | 1.67 ± 0.31 | 7.71 |
| | | 2 | Clear, colorless | Clear, colorless | Clear, colorless | 4.93 ± 0.99 | 0.60 ± 0.20 | 7.76 |
| | Grifols® | 1 | Clear, colorless | Clear, colorless | Clear, colorless | 5.47 ± 1.45 | 0.60 ± 0.20 | 7.61 |
| | | 2 | Clear, colorless | Clear, colorless | Clear, colorless | 4.13 ± 1.33 | 0.40 ± 0.53 | 7.63 |

Analysis of the manufactured samples in different IV bags incubated at room temperature and refrigerated conditions.

| **Sample** | **IV bag** | | **4°C** | | | | | | | |
|---|---|---|---|---|---|---|---|---|---|---|
| | | | **Visual Analysis** | | | **pH Analysis** | | | | |
| | | **Time Zero** | **12 days** | **32 days** | **3 Months** | **Time Zero** | **1 month** | | **3 months** | |
| | | | | | | **1** | **1** | **2** | **1** | **2** |
| 10 mg/mL IBU:Na₃PO₄ (1.0:0.9) | IntraVia® | Clear, colorless | Clear, colorless | Clear, colorless | Clear, colorless | 7.42 | 7.49 | 7.46 | 7.47 | 7.47 |
| | Technoflex® | Clear, colorless | Clear, colorless | Clear, colorless | Clear, colorless | 7.44 | 7.48 | 7.47 | 7.46 | 7.48 |
| | | **Time Zero** | **2 days** | **22 days** | **3 Months** | | | | | |
| | PAB® | Clear, colorless | Clear, colorless | Clear, colorless | Clear, colorless | 7.48 | 7.69 | 7.67 | 7.49 | 7.45 |
| | | **Time Zero** | **11 days** | **30 days** | **3 Months** | | | | | |
| | Viaflex® | Clear, colorless | Clear, colorless | Clear, colorless | Clear, colorless | 7.58 | 7.57* | 7.58* | 7.65 | 7.58 |
| | | **Time Zero** | **7 days** | **30 days** | **3 Months** | | | | | |
| | Grifols® | Clear, colorless | Clear, colorless | Clear, colorless | Clear, colorless | 7.36 | 7.66 | 7.63 | 7.66 | 7.63 |

| | | | | | | | | | | |
|---|---|---|---|---|---|---|---|---|---|---|
| *2 week time point | | | | | | | | | | |

| **Sample** | **IV bag** | | **4°C** | | | | | | | |
|---|---|---|---|---|---|---|---|---|---|---|
| | | | **Particle Analysis** | | | **pH Analysis** | | | | |
| | | **TimeZero** | | **3 Month** | | **Time Zero** | **1 month** | | **3 months** | |
| | | **10 µm** | **25 µm** | **10 µm** | **25 µm** | **1** | **1** | **2** | **1** | **2** |
| 10 mg/mL IBU:Na₃PO₄ | IntraVia® | 5.67 ± 1.67 | 0.40 ± 0.35 | - | - | 7.42 | 7.49 | 7.46 | 7.47 | 7.47 |
| (1.0:0.9) | Technoflex® | 2.60 ± 0.40 | 0.67 ± 0.12 | 6.53 ± 0.90 | 0.40 ± 0.20 | 7.44 | 7.48 | 7.47 | 7.46 | 7.48 |
| | PAB® | 2.33 ± 0.81 | 0.27 ± 0.12 | 22.0 ± 0.87 | 1.60 ± 0.87 | 7.48 | 7.69 | 7.67 | 7.49 | 7.45 |
| | Viaflex® | 0.67 ± 0.31 | 0.07 ±0.12 | 1.07 ± 0.31 | 0.13 ± 0.12 | 7.58 | 7.57* | 7.58* | 7.65 | 7.58 |
| | Grifols® | 1.33 ± 0.61 | 0.27 ± 0.23 | 2.87 ± 0.61 | 0.40 ± 0.0 | 7.36 | 7.66 | 7.63 | 7.66 | 7.63 |

| | | | | | | | | | | |
|---|---|---|---|---|---|---|---|---|---|---|
| * 2 week time point | | | | | | | | | | |

| **Sample** | **IV bag** | **25°C** | | | | **25°C** | | | | |
|---|---|---|---|---|---|---|---|---|---|---|
| | | | **Visual Analysis** | | | **pH Analysis** | | | | |
| | | **Time Zero** | **12 days** | **34 days** | **3 Months** | **Time Zero** | **1 Month** | | **3** | **Months** |
| | | | | | | | **1** | **2** | **1** | **2** |
| 10 mg/mL IBU:Na₃PO₄ | IntraVia® | Clear, colorless | Clear, colorless | Clear, colorless | Clear, colorless | 7.42 | 7.55 | 7.57 | 7.58 | - |
| (1.0:0.9) | Technoflex® | Clear, colorless | Clear, colorless | Clear, colorless | Clear, colorless | 7.44 | 8.08 | 8.06 | 7.65 | 7.55 |
| | | **Time Zero** | **2 days** | **24 days** | **3 Months** | | | | | |
| | PAB® | Clear, colorless | Clear, colorless | Clear, colorless | Clear, colorless | 7.48 | 7.47 | 7.45 | 7.48 | 7.48 |
| | | **Time Zero** | **13 days** | **-** | **3 Months** | | | | | |
| | Viaflex® | Clear, colorless | Clear, colorless | - | Clear, colorless | 7.58 | 7.91* | 7.92* | 8.21 | 8.18 |
| | | **Time Zero** | **7 days** | **30 days** | **3 Months** | | | | | |
| | Grifols® | Clear, colorless | Clear, colorless | Clear, colorless | ^{τ}Clear, colorless | 7.36 | 7.66 | 7.63 | 7.47 | 7.47 |

| | | | | | | | | | | |
|---|---|---|---|---|---|---|---|---|---|---|
| * 2 week time point τ One sample had some visible precipitate. | | | | | | | | | | |

| | | | **Incubated at 50°C** | | |
|---|---|---|---|---|---|
| **Sample** | **IV bag** | | **Appearance** | | |
| | | Sample | **Time Zero** | **1 Month** | **3 Month** |
| 10 mg/mL IBU:Na₃PO₄ | IntraVia® | 1 | Clear, colorless | Clear, colorless | Sample Evaporated |
| (1.0:0.9) | Technoflex ® | 1 | Clear, colorless | Clear, colorless | Clear, colorless |
| | PAB® | 1 | Clear, colorless | Clear, colorless | Clear, colorless |
| | Viaflex® | 1 | Clear, colorless | Sample Evaporated | - |
| | Grifols® | 1 | Clear, colorless | Clear. Colorless | Clear, colorless |

Analysis of the manufactured samples in different IV bags after autoclaving.

| **Sample** | **IV bag** | **Autoclaved** | | **Particle Analysis** | | **pH** |
|---|---|---|---|---|---|---|
| | | **Before** | **After** | **10 µm** | **25 µm** | **-** |
| 10 mg/mL IBU:Na₃PO₄ | IntraVia® (2-14-12) | Clear, colorless | Clear, colorless | 27.93 ± 0.46 | 0.93 ± 0.42 | 7.52 |
| (1.0:0.9) | Technoflex® | Clear, colorless | Clear, colorless | 1.53 ± 0.50 | 0.27 ± 0. 12 | 7.73 |
| | PAB® | Clear, colorless | Clear, colorless | 19.0 ± 1.91 | 0.67 ± 0.23 | 7.83 |
| | Viaflex® | Clear, colorless | Clear, colorless | 1.80 ± 0.00 | 0.00 ± 0.00 | 8.02 |
| | Grifols® | Clear, colorless | Clear, colorless | 2.33 ± 0.58 | 0.40 ± 0.20 | 8.03 |

### Chemical Stability

**IntraVia®**

| | | | | | | | | 1 month | | | | 3 month | | | |
|---|---|---|---|---|---|---|---|---|---|---|---|---|---|---|---|
| To | | | | After Autoclaving | | | | 50°C | | | | 50°C | | | |
| RT | RRT | Area | Area% | RT | RRT | Area | Area% | RT | RRT | Area | Area% | RT | RRT | Area | Area% |
| 3.61 | 0.48 | 2.51 | 0.02 | 3.56 | 0.48 | 2.57 | 0.02 | 3.55 | 0.48 | 2.71 | 0.02 | 3.64 | 0.48 | 1.68 | 0.02 |
| 7.57 | 1.00 | 12210.40 | 99.98 | 7.44 | 1.00 | 12601.40 | 99.98 | 7.41 | 1.00 | 13442.00 | 99.96 | 7.66 | 1.00 | 7950.58 | 99.95 |
| | | | | | | | | 12.68 | 1.71 | 2.65 | 0.02 | 13.19 | 1.72 | 2.48 | 0.03 |

**Technoflex®**

| | | | | | | | | 1 month | | | | 3 month | | | |
|---|---|---|---|---|---|---|---|---|---|---|---|---|---|---|---|
| To | | | | After Autoclaving | | | | 50°C | | | | 50°C | | | |
| RT | RRT | Area | Area% | RT | RRT | Area | Area% | RT | RRT | Area | Area% | RT | RRT | Area | Area% |
| 1.55 | 0.21 | 3.55 | 0.03 | | | | | | | | | | | | |
| 3.57 | 0.48 | 2.64 | 0.02 | 3.57 | 0.48 | 2.64 | 0.02 | 3.56 | 0.48 | 2.70 | 0.02 | 3.62 | 0.48 | 1.84 | 0.02 |
| 7.44 | 1.00 | 12260.50 | 99.95 | 7.45 | 1.00 | 12658.10 | 99.98 | 7.42 | 1.00 | 14305.10 | 99.98 | 7.59 | 1.00 | 8882.27 | 99.98 |

**PAB®**

| | | | | | | | | 2 Month | | | | 3 Month | | | |
|---|---|---|---|---|---|---|---|---|---|---|---|---|---|---|---|
| To | | | | After Autoclaving | | | | 50°C | | | | 50C | | | |
| RT | RR T | Area | Area % | RT | RR T | Area | Area % | RT | RR T | Area | Area % | RT | RR T | Area | Area % |
| 3.5 6 | 0.48 | 2.20 | 0.02 | 3.5 5 | 0.48 | 2.51 | 0.02 | 3.5 9 | 0.48 | 2.52 | 0.02 | 3.6 2 | 0.48 | 2.77 | 0.02 |
| 7.4 3 | 1.00 | 11492.4 0 | 99.98 | 7.4 1 | 1.00 | 12841.9 0 | 99.98 | 7.5 0 | 1.00 | 13104.9 0 | 99.98 | 7.5 7 | 1.00 | 14289.1 0 | 99.98 |

**Viaflex®**

| To | | | | After Autoclaving | | | |
|---|---|---|---|---|---|---|---|
| RT | RRT | Area | Area% | RT | RRT | Area | Area% |
| 3.57 | 0.48 | 2.43 | 0.02 | 3.57 | 0.48 | 2.36 | 0.02 |
| 7.46 | 1.00 | 13046.10 | 99.98 | 7.45 | 1.00 | 12119.90 | 99.98 |

After incubation at 50°C for 1 month the samples completely evaporated.

**Grifols®**

| | | | | | | | | 1 Month | | | | 3 Month | | | |
|---|---|---|---|---|---|---|---|---|---|---|---|---|---|---|---|
| To | | | | After Autoclaving | | | | 50°C | | | | 50°C | | | |
| RT | RR T | Area | Area % | RT | RR T | Area | Area % | RT | RR T | Area | Area % | RT | RR T | Area | Area % |
| 3.6 1 | 0.48 | 2.38 | 0.02 | 3.6 1 | 0.48 | 2.53 | 0.02 | 3.7 1 | 0.47 | 2.68 | 0.02 | 3.6 1 | 0.48 | 3.22 | 0.02 |
| 7.5 5 | 1.00 | 12918.2 0 | 99.98 | 7.5 4 | 1.00 | 12876.0 0 | 99.98 | 7.8 2 | 1.00 | 14654.2 0 | 99.98 | 7.5 6 | 1.00 | 20306.8 0 | 99.98 |

The results show that the formulation remained clear and colorless in each of the different IV bags tested.

### EXAMPLE 5: Chemical Stability of Ibuprofen Formulations Containing Potassium Phosphate

The chemical stability of formulations at 10 mg/mL IBU with K₃PO₄ with a molar ratio of 0.82:1.0 (K₃PO₄:IBU) was determined. Samples were filled into 3 mL glass vials, stoppered with rubber septa, and capped and then either autoclaved or stored at 40 and 50°C for 1 month. The results are set forth in Table 5 below.

**Table 5: Chemical stability of 10 mg/mL IBU dissolved in K₃PO₄.**

| To | | | | After Autoclaving | | | | Incubated for 1 Month at 40 or 50°C | | | | | | | |
|---|---|---|---|---|---|---|---|---|---|---|---|---|---|---|---|
| - | | | | - | | | | 40°C | | | | 50°C | | | |
| RT | RRT | Area | Area % | R T | RRT | Area | Area % | RT | RRT | Area | Area % | RT | RRT | Area | Area% |
| | | | | 1.85 | 0.21 | 2.60 | 0.02 | | | | | | | | |
| 4.1 6 | 0.4 8 | 2.84 | 0.02 | 4.16 | 0.48 | 2.71 | 0.02 | 3.6 2 | 0.4 8 | 2.46 | 0.02 | 3.6 2 | 0.4 8 | 2.53 | 0.02 |
| 8.7 1 | 1.0 0 | 13314.70 | 99.98 | 8.71 | 1.00 | 13570.5 0 | 99.96 | 7.5 9 | 1.0 0 | 11839.0 0 | 99.98 | 7.5 9 | 1.0 0 | 11870.8 0 | 99.98 |

As can be seen from the results set forth in Table 5, there was no detectable chemical degradation for this formulation under these conditions.

The chemical stability of IBU formulations at 4, 8, and 16 mg/mL containing Na₃PO₄ with a molar ratio of 0.8:1.0 (Na₃PO₄:IBU) was determined after autoclaving samples in glass vials. The results are set forth in Table 6 below.

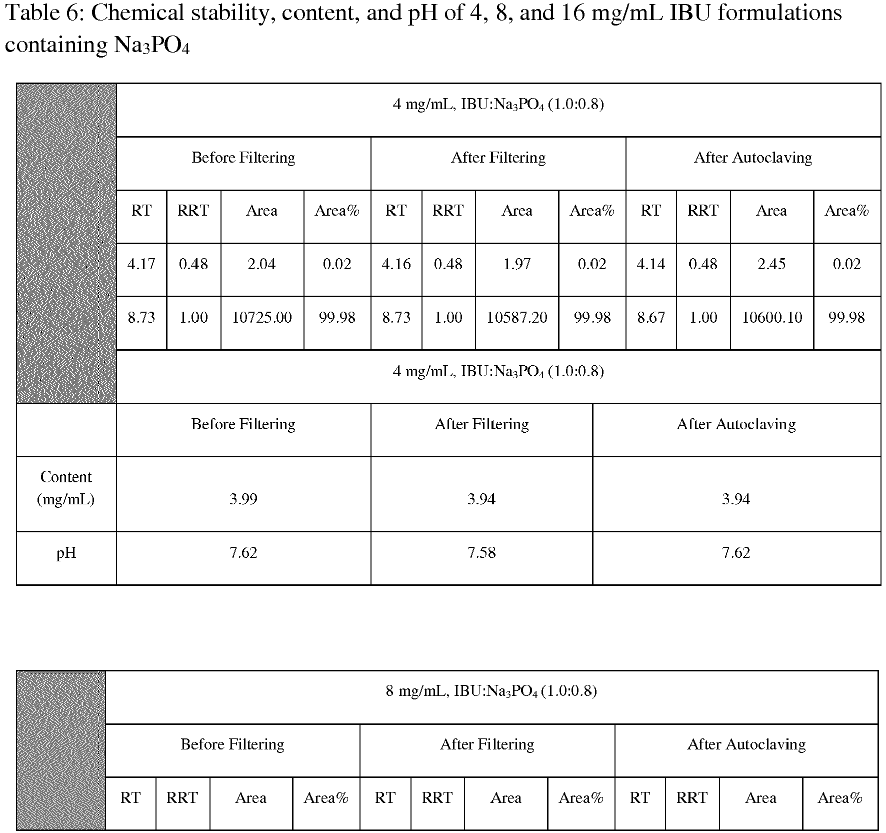

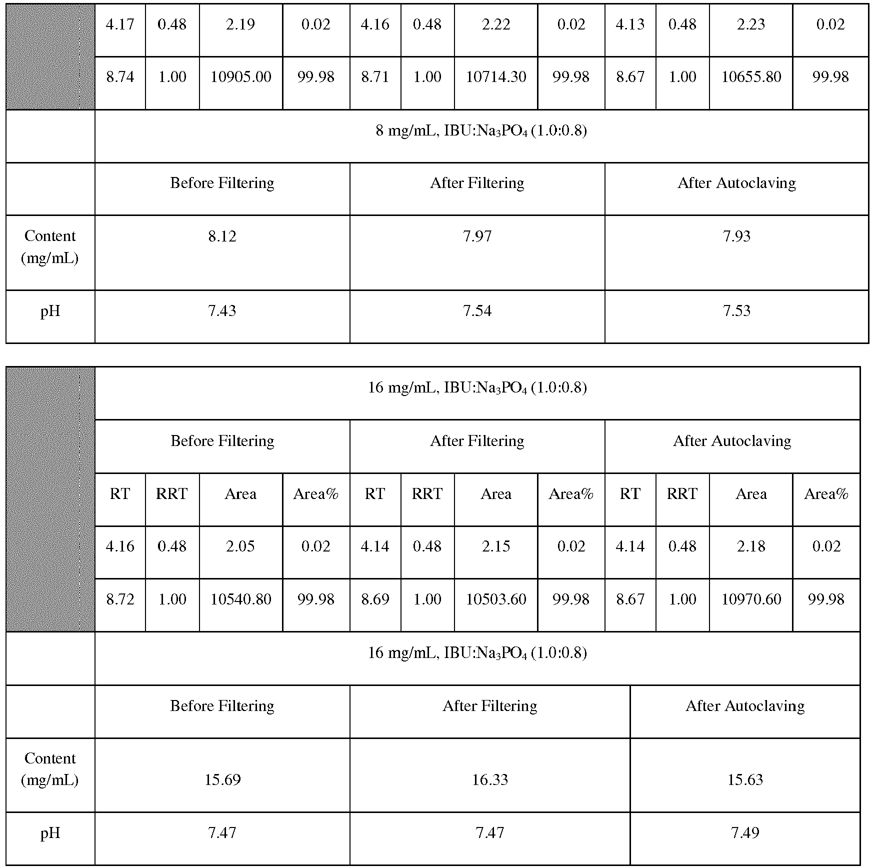

As can be seen from the results set forth in Table 6, there was no detectable chemical degradation for these formulations under these conditions.

### EXAMPLE 6 (reference) : Solubility of Ibuprofen: Sodium Hydroxide Formulations

Ibuprofen was soluble up to 100 mg/mL with Sodium Hydroxide (NaOH) at molar ratios of 0.95:1.0, 1.0:1.0, and 1.05:1.0 (NaOH:IBU). Upon sample dilution the solution became a cloudy white precipitate at molar ratios of 0.95:1.0 and 1.0:1.0 (Table 7). When the IBU sample at the 1.05:1.0 (NaOH:IBU) molar ratio was diluted to lower concentrations, the solution was clear and colorless at each concentration (Table 2).

**Table 7: Solubility of IBU with NaOH**

| | NaOH:IBU | | | | | |
|---|---|---|---|---|---|---|
| Molar Ratio | 0.95:1.00 | | 1.00:1.00 | | 1.05:1.00 | |
| Concentration (mg/mL) | Appearance | pH | Appearance | pH | Appearance | pH |
| 100 | Clear, colorless | 7.35 | Clear, colorless | 7.61 | Clear, colorless | 10.09 |
| 25 | Clear, colorless | 7.05 | Cloudy White ppt | 7.09 | Clear, colorless | 9.81 |
| 10 | Cloudy white haze | 6.73 | Cloudy White ppt | 6.77 | Clear, colorless | 9.75 |
| 8 | Cloudy white haze | 6.71 | Chunky White ppt | 6.70 | Clear, colorless | 9.71 |
| 4 | white haze | 6.44 | White ppt | 6.48 | Clear, colorless | 9.53 |
| 3.2 | white haze | 6.35 | White ppt | 6.34 | Clear, colorless | 9.43 |

### EXAMPLE 7 (reference) : Solubility of Ibuprofen: L-Lysine Formulations

Ibuprofen solutions were made at concentrations of 3.2, 4.0, and 8.0 mg/mL with L-Lysine (Lys) base. Ibuprofen was able to be dissolved at these concentrations only at a molar ratio of ∼ 1.0:1.0 (Lys:IBU) (Table 8).

**Table 8: Solubility of IBU with Lys.**

| IBU dissolved with L-Lysine | | | |
|---|---|---|---|
| IBU Conc. (mg/mL) | Lys:IBU Final Molar Ratio | Appearance | pH |
| 3.2 | 1.019:1.000 | Clear and colorless | 6.83 |
| 4.0 | 1.003:1.000 | Clear and colorless | 6.84 |
| 8.0 | 0.995:1.000 | Clear and colorless | 6.83 |

### EXAMPLE 8 (reference) : Solubility of Ibuprofen with Sodium and Potassium Carbonate

Ibuprofen was dissolved at 100 mg/mL with both potassium and sodium carbonate at molar ratios ranging from 0.5:1.0 to 0.9:1.0 (Na₂CO₃/K₂CO₃:IBU). The solutions were clear and colorless at all concentrations tested when the molar ratio was 0.6:1.0 to 0.9:1.0 using either Na₂CO₃ (Table 9) or K₂CO₃ (Table 10).

**Table 9: Solubility of IBU with Na₂CO₃**

| | Na₂CO₃:IBU | | | | | | | | | |
|---|---|---|---|---|---|---|---|---|---|---|
| Molar Ratio | 0.5:1.0 | | 0.6:1.0 | | 0.7:1.0 | | 0.8:1.0 | | 0.9:1.0 | |
| Concentration (mg/mL) | Appearance | pH | Appearance | pH | Appearance | pH | Appearance | pH | Appearan ce | pH |
| 100 | Clear, colorless | 7.56 | Clear, colorless | 7.50 | Clear, colorless | 7.69 | Clear, colorless | 8.01 | Clear, colorless | 8.11 |
| 25 | White ppt | 7.14 | Clear, colorless | 7.72 | Clear, colorless | 8.29 | Clear, colorless | 8.45 | Clear, colorless | 8.54 |
| 10 | White Chunky ppt | 6.90 | Clear, colorless | 7.93 | Clear, colorless | 8.46 | Clear, colorless | 8.59 | Clear, colorless | 8.69 |
| 8 | White Chunky ppt | 6.76 | Clear, colorless | 8.00 | Clear, colorless | 8.51 | Clear, colorless | 8.62 | Clear, colorless | 8.72 |
| 4 | White ppt | 6.56 | Clear, colorless | 8.05 | Clear, colorless | 8.57 | Clear, colorless | 8.69 | Clear, colorless | 8.79 |
| 3.2 | Very Slight white ppt | 6.50 | Clear, colorless | 8.09 | Clear, colorless | 8.58 | Clear, colorless | 8.71 | Clear, colorless | 8.81 |

**Table 10: Solubility of IBU with K₂CO₃.**

| | K₂CO₃:IBU | | | | | | | | | | | |
|---|---|---|---|---|---|---|---|---|---|---|---|---|
| Molar Ratio | 0.5:1.0 | | 0.6:1.0 | | 0.7:1.0 | | 0.8:1.0 | | 0.9:1.0 | | 1.0:1.0 | |
| Concentration (mg/mL) | Appearance | pH | Appearance | pH | Appearance | pH | Appearance | pH | Appearance | pH | Appearance | pH |
| 100 | Clear, colorless | 7.37 | Clear, colorless | 7.49 | Clear, colorless | 7.43 | Clear, colorless | 7.74 | Clear, colorless | 8.23 | Clear, colorless | 8.57 |
| 25 | White haze, ppt | 6.90 | Clear, colorless | 8.02 | Clear, colorless | 8.18 | Clear, colorless | 8.28 | Clear, colorless | 8.45 | Clear, colorless | 8.79 |
| 10 | White haze, ppt | 6.80 | Clear, colorless | 8.15 | Clear, colorless | 8.29 | Clear, colorless | 8.50 | Clear, colorless | 8.57 | Clear, colorless | 8.90 |
| 8 | White haze, ppt | 6.78 | Clear, colorless | 8.18 | Clear, colorless | 8.32 | Clear, colorless | 8.52 | Clear, colorless | 8.60 | Clear, colorless | 8.92 |
| 4 | White haze | 6.56 | Clear, colorless | 8.24 | Clear, colorless | 8.39 | Clear, colorless | 8.58 | Clear, colorless | 8.67 | Clear, colorless | 8.98 |
| 3.2 | White haze | 6.44 | Clear, colorless | 8.25 | Clear, colorless | 8.40 | Clear, colorless | 8.59 | Clear, colorless | 8.68 | Clear, colorless | 9.00 |

While Na₂CO₃ displayed useful properties as a solubilizer for ibuprofen, a 10 mg/mL IBU formulation with Na₂CO₃ became hazy and displayed undesirable pH shifts during stability testing.

### COMPARATIVE EXAMPLE A

In Comparative Example A, Megulmine was used as a solubilizer for ibuprofen. Ibuprofen was soluble with Meglumine (Meg) at 100 mg/mL when made at a molar ratio of 0.9:1.0 (Meg:IBU). When the sample was diluted to lower concentrations the clear and colorless solution became hazy and then formed a cloudy white precipitate (Table 11).

**Table 11: Solubility of IBU with Meg**

| | Meg:IBU (Molar Ratio, 0.90:1.00) | |
|---|---|---|
| Concentration (mg/mL) | Appearance | pH |
| 100 | Clear and colorless | 7.19 |
| 25 | White Haze | 6.62 |
| 10 | Cloudy white ppt | 6.67 |
| 8 | Cloudy white ppt | 6.63 |
| 4 | Cloudy White ppt | 6.38 |
| 3.2 | Cloudy White ppt | 6.30 |

### COMPARATIVE EXAMPLES B - E

In Comparative Examples B - E, other potential ibuprofen solubilizing agents were utilized to form aqueous solutions of ibuprofen at a molar ratio of solubilizing agent to ibuprofen of 0.90 to 1.0. In Comparative Example B, sodium bicarbonate was used as the ibuprofen solubilizing agent. In Comparative Example C, sodium citrate dihydrate was used. In Comparative Example D, dibasic potassium phosphate was used as the ibuprofen solubilizing agent. Sodium bicarbonate, sodium citrate dihydrate, and dibasic potassium phosphate were unable to dissolve Ibuprofen at 100 mg/mL using a molar ratio of 0.9 to 1.0 of base/buffer to IBU. In Comparative Example E, trisodium citrate was used as the ibuprofen solubilizing agent. Trisodium citrate was unable to dissolve Ibuprofen at 50 mg/mL IBU with 0.9:1.0 Trisodium Citrate:IBU. The sample contained a significant amount of precipitate which remained even when the ratio was increased to 1:1.

### COMPARATIVE EXAMPLE F

In Comparative Example F, diethanolamine was utilized as an ibuprofen solubilizing agent to form aqueous solutions of ibuprofen. The ibuprofen was solubilized at 100mg/mL at a molar ratio of ibuprofen solubilizing agent to ibuprofen of 0.95 to 1.0 (pH = 7.00). Thereafter, the ibuprofen diluted. The results are provided in Table 12 below:

**Table 12**

| Dilution (mg/mL) | Appearance | pH |
|---|---|---|
| 25 | Cloudy white haze | 6.72 |
| 10 | Bright White ppt | 6.23 |
| 8 | Cloudy white ppt | 6.68 |
| 4 | Cloudy white ppt | 6.39 |
| 3.2 | Cloudy White ppt | 6.30 |

Triethanolamine was able to solubilize ibuprofen at a molar ratio of approximately 1:1 but when diluted it also precipitated like diethanolamine. It is hypothesized that monoethanolamine may behave similarly.

### COMPARATIVE EXAMPLE G

Formulations containing the monobasic sodium phosphate:ibuprofen molar ratios at the high end and low end of the range as described in the Zhang reference (i.e., 0.43:1.0 and 1.19:1.0 NaH2PO4.H2O:IBU) were prepared at concentrations of 100 mg/mL ibuprofen (IBU), tested and were then diluted to a concentration as low as 5 mg/mL IBU. Visual and pH analyses were performed, as per the following:

### Materials:

IBU = 206.30 g/mol
NaH2PO4.H2O= 137.99 g/mol
WFI
250 mL stainless steel container, Magnetic Stir bar

### Procedure:

1. Try to make 0.43:1.0 (NaH2PO4.H2O:IBU) at 100 mg/mL. Add 1,439 g of NaH2PO4.H2O and 5.00g IBU, q.s. to 50 mLs with WFI.
2. Try to make 1.18:1.0 (NaH2PO4.H2O:IBU) at 100 mg/mL. Add 3.947 g of NaH2PO4.H2O and 5.00 g IBU, q.s. to 50 mLs with WFI. ("IBU" means ibuprofen; "WFI" means water for injection).

The results are set forth in the Table 13 below:

**Table 13**

| | 0.43:1.0 (NaH₂PO₄.H₂O:IBU) | | 1.18:1.0 (NaH₂PO₄.H₂O:IBU) | |
|---|---|---|---|---|
| Concentration (mg/mL) | Visual Analysis | pH | Visual Analysis | pH |
| 100 | Huge White Precipitate | 4.28 | Huge White Precipitate | 4.31 |
| 50 | Huge White Precipitate | 4.38 | Huge White Precipitate | 4.34 |
| 25 | Huge White Precipitate | 4.38 | Huge White Precipitate | 4.40 |
| 10 | Huge White Precipitate | 4.29 | Huge White Precipitate | 4.37 |
| 5 | Huge White Precipitate | 4.28 | Huge White Precipitate | 4.37 |

As can be seen from Table 13, the aqueous ibuprofen product prepared at the low end of the molar ratio of monobasic sodium phosphate: ibuprofen described in the Zhang reference (0.43:1) and at the high end of the molar ratio of monobasic sodium phosphate:ibuprofen (1.18:1) provided a solution with a huge white precipitate. Injectable formulations should not have insoluble, particulate matter contained therein, and therefore these ibuprofen formulations do not provide an acceptable product.

### EXAMPLE 9

The objective of Example 9 was to assess the acute irritation potential of aqueous solutions of ibuprofen using sodium phosphate and potassium phosphate as ibuprofen solubilizing agents when injected intravenously into rabbits.

The test articles for this study were as follows:
- Test Article 1: Caldolor® (Ibuprofen) Injection
- Test Article 2: Ibuprofen (5 mg/mL) in Na₃PO₄
- Test Article 3: Ibuprofen (10 mg/mL) in Na₃PO₄
- Test Article 4: Ibuprofen (80 mg/mL) in Na₃PO₄
- Test Article 5: Ibuprofen (10 mg/mL) in K₃PO₄.

Rabbits were divided into in Groups 1-6, and were dosed once by slow bolus intravenous injection over approximately 60 seconds into the ear vein. Animals were dosed at concentrations of 1mL/kg (Vehicle), 4 mg/mL (Test Article 1), 5 mg/mL (Test Article 2), 10 mg/mL (Test Articles 3 and 5), and 80 mg/mL (Test Article 4). The molar ratio of Na₃PO₄ and K₃PO₄ to ibuprofen in this study was 0.9:1 and also contained sodium chloride to make an isoosmotic solution. Animals were euthanized by intravenous barbiturate overdose following the final skin/dermal grading.

Table 14 below reflects the study design as it pertained to the pathology aspect of this study.

**Table 14**

| Group | Number/Group | Dose Concentration | | Sacrifice Time (hr) |
|---|---|---|---|---|
| | | Left Ear* | Right Ear* | |
| 1 | 3 | 4 mg/mL | 5 mg/mL | 1 |
| | | Test Article 1 | Test Article 2 | |
| 2 | 3 | 4 mg/mL | 5 mg/mL | 24 |
| | | Test Article 1 | Test Article 2 | |
| 3 | 3 | 10 mg/mL | 10 mg/mL | 1 |
| | | Test Article 5 | Test Article 3 | |
| 4 | 3 | 10 mg/mL | 10 mg/mL | 24 |
| | | Test Article 5 | Test Article 3 | |
| 5 | 3 | 80 mg/mL | 1 mL/kg | 1 |
| | | Test Article 4 | Vehicle | |
| 6 | 3 | 80 mg/mL | 1 mL/kg | 24 |
| | | Test Article 4 | Vehicle | |

| | | | | |
|---|---|---|---|---|
| * Dose Volume 1 mL/kg administered over approximately 60 seconds. | | | | |

No gross necropsy was performed at in-life termination. After the animals were euthanized, a full thickness section (approximately 6 x 6 cm) of the pinna of both ears, including the artery and the site of injection, were excised and fixed in 10% neutral buffered formalin for histopathological evaluation.

Table 15 below provides draize scoring with erythema and edema scores at 1 hour post injection (Groups 1, 3 and 5) and at 1 and 24 hours post injection (Groups 2 and 4).

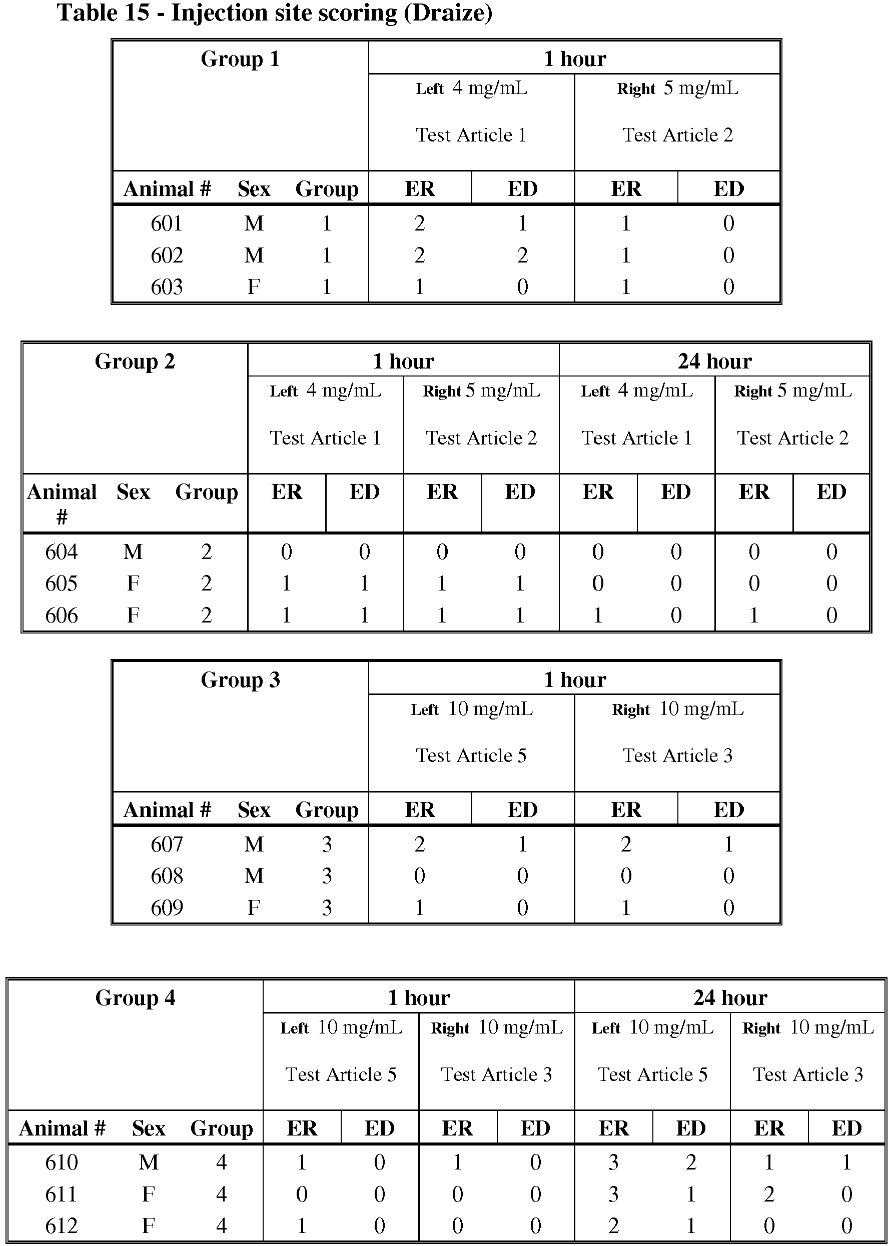

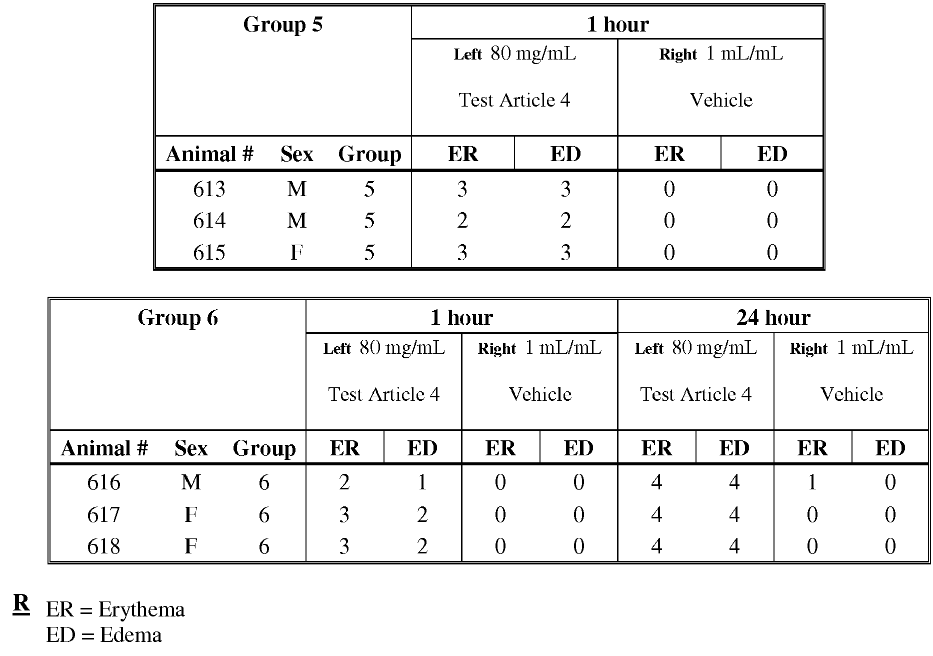

### Injection with Vehicle (Groups 5 and 6, right ear)

At one hour, animals injected with the vehicle exhibited hemorrhage, vascular necrosis and edema. At 24 hours, animals injected with the vehicle were essentially normal, with only one animal exhibiting moderate edema. Lesions at one hour are associated with the mechanical aspect of injection.

### Test Article 1 - Caldolor® (Ibuprofen) Injection (Groups 1 and 2, left ear)

At one hour, only one animal exhibited microscopic change, that being vascular necrosis and hemorrhage. At 24 hours, all animals were microscopically normal, indicating that the change at one hour was most likely associated with the mechanical aspect of injection.

### Test Article 2: Ibuprofen (5 mg/mL) in Na₃PO₄ (Groups 1 and 2, right ear)

At one hour, all animals exhibited edema. One also exhibited minimal vascular necrosis, while a second had a minimal mixed cell inflammation composed of 100% mononuclear cells. At 24 hours, two of three animals only exhibited slight to minimal hemorrhage, while the third had moderate vascular necrosis with mild hemorrhage and a minimal inflammatory response composed of 80% neutrophils.

### Test Article 3: Ibuprofen (10 mg/mL) in Na₃PO₄ (Groups 3 and 4, right ear)

At one hour, only one animal exhibited a microscopic change, that being mild edema. At 24 hours, one animal exhibited mild edema and a second animal exhibited mild hemorrhage.

### Test Article 4: Ibuprofen (80 mg/mL) in Na₃PO₄ (Groups 5 and 6, left ear)

At one hour, one animal exhibited mild edema, mild vascular necrosis and mild hemorrhage. At 24 hours, all three animals exhibited mild vascular necrosis with mild to moderate inflammation, comprised of 90 or 100% neutrophils. Moderate hemorrhage was noted in all animals.

### Test Article 5: Ibuprofen (10 mg/mL) in K₃PO₄ (Groups 3 and 4, left ear)

At one hour, two animals exhibited mild to moderate vascular necrosis with a corresponding mild to moderate hemorrhage. The third animal of the group exhibited mild edema. At 24 hours, one animal was normal, one exhibited only minimal vascular necrosis, while the third had mild edema, mild vascular necrosis, mild hemorrhage and a minimal inflammatory infiltrate comprised of 80% neutrophils.

### Summary of Results

The results from Table 14 show that the current commercially available product (Caldolor®) diluted to 4mg/mL (as is done when the product is infused into patients) is similar to the sodium phosphate (Na₃PO₄) 5mg/mL and 10mg/mL formulations at 1 and 24 hours. The potassium phosphate (K₃PO₄) formulation at 10mg/mL (test article 5) showed a greater amount of erythema at 24 hours. The undiluted sodium phosphate formulation at 80mg/mL showed more erythema and edema at 1 and 24 hours. The current formulation (Caldolor®) at 4mg/mL, the 5mg/mL and 10mg/mL sodium phosphate and vehicle all provided similar results. The potassium phosphate formulation at 10mg/mL was somewhat more irritating. The undiluted 80mg/mL sodium phosphate formulation the most irritating.

### EXAMPLE 10

The objective of Example 10 was to make ibuprofen solutions with a combination of sodium phosphate dibasic and sodium hydroxide (NaOH) and test whether clear and colorless solutions of 10 mg/mL Ibuprofen (IBU) can be made at molar ratios of 0.7:1.0, 0.8:1.0, and 0.9:1.0 (Sodium Phosphate dibasic [Na₂HPO₄]:IBU).

### Materials:

IBU = 206.30 g/mol
Na₂HPO₄ = 141.96 g/mol
WFI (water for injection)
1 N Solution of NaOH
250 mL stainless steel container, Magnetic Stir bar

### Procedure:

1. Solution samples of 10 mg/mL IBU at molar ratios of 0.5:1.0, 0.70:1.0, 0.8:1.0, and 0.9:1.0 (Na₂HPO₄:IBU) were made. The pH of these solutions were adjusted with NaOH with different order of additions.
   A. 200 mLs of 0.5:1.0 (Na₂HPO₄:IBU) Add 0.688 g Na₂HPO₄, adjust to pH 12.0 with 1 N NaOH. Add 2.0 grams of IBU. Mix and then q.s. to volume with WFI.
   B. 200 mLs of 0.7:1.0 (Na₂HPO₄:IBU). Add 0.963 g of Na₂HPO₄, adjust to pH 11.0 with 1 N NaOH. Add 2.0 grams of IBU. Mix and then q.s. to volume with WFI.
   C. 200 mLs of 0.8:1.0 (Na₂HPO₄:IBU). Add 1.101 g of Na₂HPO₄. Add 2.0 grams of IBU. Adjust the pH with NaOH. Mix and then q.s. to volume with WFI.
   D. 200 mLs of 0:7:1.0 (Na₂HPO₄:IBU). Add 0.936 g of Na₂HPO₄, adjust to pH 12.0 with 1 N NaOH. Add 2.0 grams of IBU. Mix and then q.s. to volume with WFI.
   E. 200 mLs of 0.8:1.0 (Na₂HPO₄:IBU). Add 1.101 g of Na₂HPO₄, adjust to pH 12.0 with 1 N NaOH. Add 2.0 grams of IBU. Mix and q.s. to volume with WFI.
   F. 200 mLs of 0.9:1.0 (Na₂HPO₄:IBU). Add 1.238 g of Na₂HPO₄, adjust to pH 12.0 with 1 N NaOH. Add 2.0 grams of IBU. Mix and q.s. to volume with WFI.
2. The pH and visual appearance of each solution at room temperature was recorded.

### Results:

Clear and colorless solutions of IBU were obtained when it was dissolved into solutions containing Na₂HPO₄ adjusted to pH 12.0 with NaOH (Table 16, Sample E and F). IBU completely dissolved at 10 mg/mL when the final molar ratio of Na₂HPO₄ to IBU was either 0.8:1.0 or 0.9:1.0. Using a molar ratio of 0.5:1.0 or 0.7:1.0 of Na₂HPO₄ to IBU resulted suspensions containing white chunky precipitate (Table 16, Sample A, B, and D). Also, if Na₂HPO₄ and IBU were first added together and then adjusted the pH with NaOH the solution did not become clear and colorless (Table 16, Sample C). The pH of the 0.8:1.0 and 0.9:1.0 Na₂HPO₄:IBU solutions (Samples E and F) were 7.07 and 7.43, respectively. These values were similar to 10 mg/mL IBU solutions made with Na₃PO₄ at molar ratios of 0.8:1.0 and 0.9:1.0 (Na₃PO₄:IBU) at pH 7.23 and 7.61, respectively. The osmolarity of the 0.9:1.0 Na₂HPO₄:IBU solution was also similar to a 10 mg/mL IBU solution at 0.9:1.0 Na₃PO₄:IBU. The values were 200 and 215 mOsm, respectively

**Table 16. IBU mixtures with Na₂HPO₄ and NaOH**

| Samples | Formulation | Final Molar Ratio (Na₂HPO₄/NaOH:IBU) | Visual | pH |
|---|---|---|---|---|
| A | 0.5:1.0 | 0.93:1.0 | White chunky ppt | 6.92 |
| | (Na₂HPO₄:IBU). Add Na₂HPO₄, adjust to pH 12.0 with NaOH then add in IBU | | | |
| B | 0.7:1.0 (Na₂HPO₄:IBU). Add Na₂HPO₄, adjust to pH 11.0 with NaOH then add in IBU | 0.90:1.0 | White chunky ppt | 6.92 |
| C | 0.8:1.0 | 1.01:1.0 | White chunky ppt | 6.84 |
| | (Na₂HPO₄:IBU). Add in Na₂HPO₄ and IBU first, then adjust the pH with NaOH. | | | |
| D | 0:7.0 | 1.29:1.0 | White ppt, a little clearer | 7.03 |
| | (Na₂HPO₄:IBU) Add Na₂HPO₄, adjust to pH 12.0 with NaOH then add in IBU | | | |
| E | 0.8:1.0 | 1.47:1.0 | Clear and colorless | 7.07 |
| | (Na₂HPO₄:IBU) Add Na₂HPO₄, adjust to pH 12.0 with NaOH then add in IBU | | | |
| F | 0.9:1.0 | 1.67:1.0 | Clear and colorless | 7.43 |
| | (Na₂HPO₄:IBU) Add Na₂HPO₄, adjust to pH 12.0 with NaOH then add in IBU | | | |

### EXAMPLE 11

Additional tribasic sodium phosphate:ibuprofen formulations were made having a molar ratio of 1:1 and 1.1:1, as per the following:

### Materials:

IBU = 206.30 g/mol
Na₃PO₄ = 163.94 g/mol, N
WFI
250 mL stainless steel container, Magnetic Stir bar

### Procedure:

1. Try to make 1.0:1.0 (Na₃PO₄:IBU) at 100 mg/mL. 3.974 g Na₃PO₄ and 5.00 grams of IBU, q.s. to 50 mLs.
2. Try to make 1.1:1.0 (Na₃PO₄:IBU) at 100 mg/mL. 4.371 g Na₃PO₄ and 5.00 grams of IBU, q.s. to 50 mLs.

### 1. The results are set forth in the Table 17 below:

**Table 17**

| | 1.0:1.0 Na₃PO₄:IBU | | 1.1:1.0 Na₃PO₄:IBU | |
|---|---|---|---|---|
| Concentration (mg/mL) | Visual | pH | Visual | pH |
| 100 | Visible flakes | 7.97 | White precipitate | 9.31 |
| 50 | Clear, colorless solution | 7.94 | Clear, colorless solution | 9.41 |
| 25 | Clear, colorless solution | 7.88 | Clear, colorless solution | 9.51 |
| 10 | Clear, colorless solution | 7.97 | Clear, colorless solution | 9.66 |
| 5 | Clear, colorless solution | 8.04 | Clear, colorless solution | 9.74 |

As can be seen from Table 17, the aqueous ibuprofen product prepared at molar ratios of tribasic sodium phosphate: ibuprofen of 1:1 and 1.1:1 provided visible flakes at an ibuprofen concentration of 100mg/mL, at or exceeding the solubility of tribasic sodium phosphate.

## Claims

1. A pharmaceutical composition comprising an aqueous solution of water for injection, ibuprofen and an ibuprofen solubilizing agent selected from
(i) tribasic sodium phosphate, tribasic potassium phosphate, or a combination of tribasic sodium phosphate and tribasic potassium phosphate, in a molar ratio of ibuprofen solubilizing agent to ibuprofen from 0.65:1 to 0.9:1; or
(ii) dibasic sodium phosphate, dibasic potassium phosphate, monobasic sodium phosphate, monobasic potassium phosphate, or a combination of any of the foregoing, together with an alkalinizing agent in an amount suitable to provide a solubilized ibuprofen solution when ibuprofen is added to the ibuprofen solubilizing agent/alkalanizing agent mixture in a ratio of ibuprofen solubilizing agent to ibuprofen from 0.8 to 1.1:1, such that the pH of the resultant aqueous ibuprofen solution is from 6.5 to 9;
the aqueous solution remains clear and colorless when the ibuprofen is diluted from a concentration from concentration of 100mg/ml to 1mg/ml.

2. The pharmaceutical composition of claim 1, wherein the ibuprofen solubilizing agent is tribasic sodium phosphate, tribasic potassium phosphate, or a combination of tribasic sodium phosphate and tribasic potassium phosphate, in a molar ratio of ibuprofen solubilizing agent to ibuprofen from 0.7:1 to 0.9:1.

3. The pharmaceutical composition of claim 2, wherein the ibuprofen solubilizing agent is tribasic sodium phosphate.

4. The pharmaceutical composition of claim 1, wherein the ibuprofen solubilizing agent is dibasic sodium phosphate, dibasic potassium phosphate, monobasic sodium phosphate, monobasic potassium phosphate, or a combination of any of the foregoing, together with an alkalinizing agent in an amount suitable to provide a solubilized ibuprofen solution when ibuprofen is added to the ibuprofen solubilizing agent/alkalanizing agent mixture in a ratio of ibuprofen solubilizing agent to ibuprofen from 0.8 to 0.9:1, such that the pH of the resultant aqueous ibuprofen solution is from 6.5 to 9.

5. The pharmaceutical composition of claim 4, wherein the pH of the solution prior to the addition of ibuprofen is from pH 11.5 to 12.5, is at least pH 12.

6. The pharmaceutical composition of claims 4 or 5, wherein the ibuprofen solubilizing agent is dibasic sodium phosphate and the alkalinizing agent is sodium hydroxide.

7. The pharmaceutical composition of any of claims 1 to 6, which is sterile filtered or terminally sterilized.

8. The pharmaceutical composition of any one of claims 1 to 6, which remains clear and colorless when stored for at least 12 weeks at 25°C.

9. The pharmaceutical composition of any one of claims 1 to 6, wherein the pH of the aqueous solution of ibuprofen is from 7.2 to 9.0.

10. The pharmaceutical composition of any one of claims 1 to 6, wherein the aqueous solution comprises ibuprofen in a concentration of 5mg to 15 mg/ml.

11. The pharmaceutical composition of claim 10, wherein the aqueous solution contained in the bag is from 40ml to 100ml.

12. The pharmaceutical composition of claim 10, wherein the aqueous solution contained in the bag has an ibuprofen concentration of 8mg/ml to 10mg/ml and the volume of the aqueous solution is 40ml, 50ml, 80ml or 100ml.

13. The pharmaceutical composition of any one of claims 10 to 12, which is contained in a pharmaceutical container for intravenous use, the pharmaceutical container being selected from a pre-filled bag made of a pharmaceutically acceptable polymeric material, glass bottle, or plastic bottle.

14. The pharmaceutical composition of claim 13, wherein the aqueous solution is contained in a bag made from a material selected from polypropylene, polyolefin and polyvinylchloride.

15. The pharmaceutical composition of any one of claims 1 to 6 and 11 to 12, which comprises a dose of ibuprofen from 400 mg to 800 mg.

16. The pharmaceutical composition of claim 1, wherein the aqueous solution is contained in a bag for intravenous administration at an ibuprofen concentration from 5mg/ml to 15 mg/ml and an ibuprofen dose from 400 mg to 800 mg.

17. The pharmaceutical composition of claims 1, 4 or 5, wherein the alkalinizing agent is selected from alkali or alkaline-earth metal hydroxides, carbonates, bicarbonates and phosphates, sodium borate, basic salts of organic acids, preferably sodium hydroxide.

## Patentansprüche

1. Pharmazeutische Zusammensetzung, umfassend eine wässrige Lösung von Wasser für die Injektion, Ibuprofen und einem Ibuprofen-solubilisierenden Mittel ausgewählt aus
(i) dreibasigem Natriumphosphat, dreibasigem Kaliumphosphat oder einer Kombination von dreibasigem Natriumphosphat und dreibasigem Kaliumphosphat in einem molaren Verhältnis von Ibuprofen-solubilisierendes Mittel zu Ibuprofen von 0,65:1 zu 0,9:1; oder
(i) zweibasigem Natriumphosphat, zweibasigem Kaliumphosphat, einbasigem Natriumphosphat, einbasigem Kaliumphosphat oder einer Kombination beliebiger der Genannten zusammen mit einem Alkalierungsmittel in einer Menge, die geeignet ist, um eine Lösung von solubilisiertem Ibuprofen bereitzustellen, wenn Ibuprofen zu dem Ibuprofen-solubilisierendes-Mittel/Alkalierungsmittel-Gemisch in einem Verhältnis von Ibuprofen-solubilisierendes Mittel zu Ibuprofen von 0,8 bis 1,1:1 zugegeben wird, so dass der pH-Wert der erhaltenen wässrigen Ibuprofenlösung von 6,5 bis 9 beträgt;
wobei die wässrige Lösung klar und farblos bleibt, wenn das Ibuprofen von einer Konzentration von 100 mg/ml auf 1 mg/ml verdünnt wird.

2. Pharmazeutische Zusammensetzung gemäß Anspruch 1, wobei das Ibuprofen-solubilisierende Mittel dreibasiges Natriumphosphat, dreibasiges Kaliumphosphat oder eine Kombination von dreibasigem Natriumphosphat und dreibasigem Kaliumphosphat in einem molaren Verhältnis von Ibuprofen-solubilisierendes Mittel zu Ibuprofen von 0,7:1 zu 0,9:1 ist.

3. Pharmazeutische Zusammensetzung gemäß Anspruch 2, wobei das Ibuprofen-solubilisierende Mittel dreibasiges Natriumphosphat ist.

4. Pharmazeutische Zusammensetzung gemäß Anspruch 1, wobei das Ibuprofen-solubilisierende Mittel zweibasiges Natriumphosphat, zweibasiges Kaliumphosphat, einbasiges Natriumphosphat, einbasiges Kaliumphosphat oder eine Kombination beliebiger der Genannten zusammen mit einem Alkalierungsmittel in einer Menge, die geeignet ist, um eine Lösung von solubilisiertem Ibuprofen bereitzustellen, wenn Ibuprofen zu dem Ibuprofen-solubilisierendes-Mittel/Alkalierungsmittel-Gemisch in einem Verhältnis von Ibuprofen-solubilisierendes Mittel zu Ibuprofen von 0,8 bis 0,9:1 zugegeben wird, so dass der pH-Wert der erhaltenen wässrigen Ibuprofenlösung von 6,5 bis 9 beträgt, ist.

5. Pharmazeutische Zusammensetzung gemäß Anspruch 4, wobei der pH-Wert der Lösung vor dem Zugeben von Ibuprofen von pH 11,5 bis 12,5 beträgt, wenigstens pH 12 beträgt.

6. Pharmazeutische Zusammensetzung gemäß einem der Ansprüche 4 oder 5, wobei das Ibuprofen-solubilisierende Mittel zweibasiges Natriumphosphat ist und das Alkalierungsmittel Natriumhydroxid ist.

7. Pharmazeutische Zusammensetzung gemäß einem der Ansprüche 1 bis 6, die sterilfiltriert oder endsterilisiert ist.

8. Pharmazeutische Zusammensetzung gemäß einem der Ansprüche 1 bis 6, die klar und farblos bleibt, wenn sie wenigstens 12 Wochen bei 25 °C aufbewahrt wird.

9. Pharmazeutische Zusammensetzung gemäß einem der Ansprüche 1 bis 6, wobei der pH-Wert der wässrigen Lösung von Ibuprofen von 7,2 bis 9,0 beträgt.

10. Pharmazeutische Zusammensetzung gemäß einem der Ansprüche 1 bis 6, wobei die wässrige Lösung Ibuprofen in einer Konzentration von 5 mg bis 15 mg/ml umfasst.

11. Pharmazeutische Zusammensetzung gemäß Anspruch 10, wobei es sich bei der in dem Beutel enthaltenen wässrigen Lösung um 40 ml bis 100 ml handelt.

12. Pharmazeutische Zusammensetzung gemäß Anspruch 10, wobei die in dem Beutel enthaltene wässrige Lösung eine Ibuprofen-Konzentration von 8 mg/ml bis 10 mg/ml aufweist und das Volumen der wässrigen Lösung 40 ml, 50 ml, 80 ml oder 100 ml beträgt.

13. Pharmazeutische Zusammensetzung gemäß einem der Ansprüche 10 bis 12, die in einem pharmazeutischen Behälter für die intravenöse Verwendung enthalten ist, wobei der pharmazeutische Behälter ausgewählt ist aus einem vorgefüllten Beutel aus einem pharmazeutisch verträglichen Polymermaterial, einer Glasflasche und einer Kunststoffflasche.

14. Pharmazeutische Zusammensetzung gemäß Anspruch 13, wobei die wässrige Lösung in einem Beutel bestehend aus einem Material ausgewählt aus Polypropylen, Polyolefin und Polyvinylchlorid enthalten ist.

15. Pharmazeutische Zusammensetzung gemäß einem der Ansprüche 1 bis 6 und 11 bis 12, die eine Dosis von Ibuprofen von 400 mg bis 800 mg umfasst.

16. Pharmazeutische Zusammensetzung gemäß Anspruch 1, wobei die wässrige Lösung in einem Beutel für die intravenöse Verabreichung mit einer Ibuprofen-Konzentration von 5 mg/ml bis 15 mg/ml und einer Ibuprofen-Dosis von 400 mg bis 800 mg enthalten ist.

17. Pharmazeutische Zusammensetzung gemäß Ansprüchen 1, 4 oder 5, wobei das Alkalierungsmittel ausgewählt ist aus Alkali- oder Erdalkalimetallhydroxiden, -carbonaten, -bicarbonaten und -phosphaten, Natriumborat, basischen Salzen von organischen Säuren, vorzugsweise Natriumhydroxid.

## Revendications

1. Composition pharmaceutique comprenant une solution aqueuse d'eau pour injection, de l'ibuprofène et un agent de solubilisation de l'ibuprofène choisi parmi les suivants :
(i) phosphate de trisodium, phosphate de tripotassium, ou une combinaison de phosphate de trisodium et de phosphate de tripotassium, dans un rapport molaire d'agent de solubilisation d'ibuprofène sur l'ibuprofène compris entre 0,65:1 et 0,9:1, ou
(ii) hydrogénophosphate de disodium, hydrogénophosphate de dipotassium, dihydrogénophosphate de sodium, dihydrogénophosphate de potassium, ou une combinaison de n'importe lesquels des précédents, avec un agent basicifiant à une teneur adaptée à l'obtention d'une solution d'ibuprofène solubilisé lorsque l'ibuprofène est ajouté au mélange agent de solubilisation d'ibuprofène/agent basicifiant dans un rapport d'agent de solubilisation de l'ibuprofène sur l'ibuprofène compris entre 0,8 et 1,1:1, de sorte que le pH de la solution aqueuse d'ibuprofène résultante soit compris entre 6,5 et 9 ;
la solution aqueuse reste transparente et incolore lorsque l'ibuprofène est dilué depuis une concentration de 100 mg/ml à 1 mg/ml.

2. Composition pharmaceutique selon la revendication 1, où l'agent de solubilisation de l'ibuprofène est le phosphate de trisodium, le phosphate de tripotassium ou une combinaison de phosphate de trisodium et de phosphate de tripotassium, dans un rapport molaire d'agent de solubilisation d'ibuprofène sur l'ibuprofène compris entre 0,7:1 et 0,9:1.

3. Composition pharmaceutique selon la revendication 2, où l'agent de solubilisation de l'ibuprofène est le phosphate de trisodium.

4. Composition pharmaceutique selon la revendication 1, où l'agent de solubilisation de l'ibuprofène est l'hydrogénophosphate de disodium, l'hydrogénophosphate de dipotassium, le dihydrogénophosphate de sodium, le dihydrogénophosphate de potassium, ou une combinaison de n'importe lesquels des précédents, avec un agent basicifiant à une teneur adaptée à l'obtention d'une solution d'ibuprofène solubilisé lorsque l'ibuprofène est ajouté au mélange agent de solubilisation d'ibuprofène/agent basicifiant dans un rapport d'agent de solubilisation de l'ibuprofène sur l'ibuprofène compris entre 0,8 et 0,9:1, de sorte que le pH de la solution aqueuse d'ibuprofène résultante soit compris entre 6,5 et 9.

5. Composition pharmaceutique selon la revendication 4, où le pH de la solution avant ajout d'ibuprofène est compris entre 11,5 et 12,5, est d'au moins 12.

6. Composition pharmaceutique selon les revendications 4 ou 5, où l'agent de solubilisation d'ibuprofène est l'hydrogénophosphate de disodium et l'agent basicifiant est l'hydroxyde de sodium.

7. Composition pharmaceutique selon l'une quelconque des revendications 1 à 6, qui est filtrée à l'état stérile ou stérilisée dans une dernière étape.

8. Composition pharmaceutique selon l'une quelconque des revendications 1 à 6, qui reste transparente et incolore lorsqu'elle est stockée pendant au moins 12 semaines à 25 °C.

9. Composition pharmaceutique selon l'une quelconque des revendications 1 à 6, où le pH de la solution aqueuse d'ibuprofène est compris entre 7,2 et 9,0.

10. Composition pharmaceutique selon l'une quelconque des revendications 1 à 6, où la solution aqueuse comprend de l'ibuprofène à une concentration comprise entre 5 mg et 15 mg/ml.

11. Composition pharmaceutique selon la revendication 10, où le volume de solution aqueuse contenu dans la poche est compris entre 40 ml et 100 ml.

12. Composition pharmaceutique selon la revendication 10, où la solution aqueuse contenue dans la poche présente une concentration en ibuprofène comprise entre 8 mg/ml et 10 mg/ml et le volume de la solution aqueuse est de 40 ml, 50 ml, 80 ml ou 100 ml.

13. Composition pharmaceutique selon l'une quelconque des revendications 10 à 12, qui est contenue dans un récipient pharmaceutique pour utilisation intraveineuse, le récipient pharmaceutique étant choisi parmi une poche préremplie fabriquée dans un matériau polymère pharmaceutiquement acceptable, un flacon de verre ou un flacon de plastique.

14. Composition pharmaceutique selon la revendication 13, où la solution aqueuse est contenue dans une poche fabriquée dans un matériau choisi parmi le polypropylène, les polyoléfines et le polychlorure de vinyle.

15. Composition pharmaceutique selon l'une quelconque des revendications 1 à 6 et 11 à 12, qui comprend une dose d'ibuprofène comprise entre 400 mg et 800 mg.

16. Composition pharmaceutique selon la revendication 1, où la solution aqueuse est contenue dans une poche pour administration intraveineuse à une concentration d'ibuprofène comprise entre 5 mg/ml et 15 mg/ml et une dose d'ibuprofène comprise entre 400 mg et 800 mg.

17. Composition pharmaceutique selon les revendications 1, 4 ou 5, où l'agent basicifiant est choisi parmi les hydroxydes, les carbonates, les bicarbonates et les phosphates de métaux alcalins ou alcalino-terreux, le borate de sodium, les sels basiques d'acides organiques, préférentiellement l'hydroxyde de sodium.
